# EUROPEAN PATENT SPECIFICATION

(11) **EP 4 150 119 B1**
(45) Date of publication and mention of the grant of the patent: **04.12.2024**
(21) Application number: 21739195.2
(22) Date of filing: 21.05.2021
(51) Int. Cl.: C12Q 1/6883

(54) **EPIGENETIC MODIFICATION OF SELECT GENES AS MARKERS OF HYPERSOMNOLENCE**
EPIGENETISCHE MODIFIKATION AUSGEWÄHLTER GENE ALS MARKER FÜR HYPERSOMNOLENZ
MODIFICATION ÉPIGÉNÉTIQUE DE GÈNES SÉLECTIONNÉS UTILISÉS EN TANT QUE MARQUEURS DE L'HYPERSOMNOLENCE

(30) Priority: 22.05.2020 US 202063029127 P
(43) Date of publication of application: 22.03.2023
(60) Divisional application: 24205945.9
(73) Proprietor: Wisconsin Alumni Research Foundation, Madison, WI 53726 (US)
(72) Inventor: PLANTE, David Thomas, Madison, Wisconsin 53711 (US); ALISCH, Reid Spencer, Prairie du Sac, Wisconsin 53578 (US)
(74) Representative: Williams Powell
(86) International application number: PCT/US2021/033677
(87) International publication number: WO 2021/237103

(56) References cited:
- US-A1- 2013 129 668
- RUTTEN B P F ET AL: "Longitudinal analyses of the DNA methylome in deployed military servicemen identify susceptibility loci for post-traumatic stress disorder", MOLECULAR PSYCHIATRY, vol. 23, no. 5, 20 June 2017 (2017-06-20), GB, pages 1145 - 1156, XP055839100, ISSN: 1359-4184, Retrieved from the Internet <URL:http://www.nature.com/articles/mp2017120> [retrieved on 20210907], DOI: 10.1038/mp.2017.120
- LEGENDRE CHRISTOPHE R. ET AL: "Pathway Implications of Aberrant Global Methylation in Adrenocortical Cancer", PLOS ONE, vol. 11, no. 3, 10 March 2016 (2016-03-10), pages e0150629, XP055839104, DOI: 10.1371/journal.pone.0150629
- SAHAR HOUSHDARAN ET AL: "Widespread Epigenetic Abnormalities Suggest a Broad DNA Methylation Erasure Defect in Abnormal Human Sperm", PLOS ONE, vol. 2, no. 12, 12 December 2007 (2007-12-12), pages e1289, XP055353923, DOI: 10.1371/journal.pone.0001289
- SAHAR HOUSHDARAN ET AL: "Widespread Epigenetic Abnormalities Suggest a Broad DNA Methylation Erasure Defect in Abnormal Human Sperm - Table S1", PLOS ONE, vol. 2, no. 12, 12 December 2007 (2007-12-12), pages 1 - 7, XP055838757, Retrieved from the Internet <URL:https://journals.plos.org/plosone/article?id=10.1371/journal.pone.0001289#s5> [retrieved on 20210907], DOI: 10.1371/journal.pone.0001289
- BARATEAU LUCIE ET AL: "Hypersomnolence, Hypersomnia, and Mood Disorders", CURRENT PSYCHIATRY REPORTS, CURRENT SCIENCE, US, vol. 19, no. 2, 27 February 2017 (2017-02-27), pages 1 - 11, XP036176635, ISSN: 1523-3812, [retrieved on 20210907], DOI: 10.1007/S11920-017-0763-0

## Description

### FIELD OF THE INVENTION

The invention is directed to tools and methods for diagnosing and providing a prognosis of hypersomnolence, idiopathic hypersomnia, and long sleep time using differentially methylated positions.

### BACKGROUND

Disorders of unexplained hypersomnolence are a significant cause of dysfunction and reduced quality of life (Khan and Trotti. Chest. 2015, 148(1):262-273). Much of the research into disorders such as idiopathic hypersomnia (IH) has focused on symptoms of chronic daytime sleepiness and/or impaired quality of wakefulness. However, excessive quantities of sleep that are nonrestorative contribute equally if not more to the impairing aspects of these disorders. Currently, the pathophysiology that underlies excessive sleep duration *(i.e.,* hypersomnia) in disorders such as IH remains unknown and is a vital area of investigation for sleep medicine.

A key step to begin to understand the biological causes of hypersomnia requires examination of factors that contribute to habitual sleep duration more broadly. While the specific determinants of habitual sleep time remain unknown, recent genome wide association studies (GWAS) have highlighted the role of the paired box 8 (*PAX8*) gene in human sleep duration. PAX8 likely serves several roles as a master transcription factor crucial to embryonic development and maintenance functions after birth (Fernández et al. Nat Rev Endocrinol. 2015, 11(1):29-42). Using both self-report and accelerometer data in population-based cohorts, single nucleotide polymorphisms (SNPs) in *PAX8* and its related antisense gene (*PAX8-AS1*) have repeatedly demonstrated significant effects on habitual sleep duration (Gottlieb et al. Mol Psychiatry. 2015, 20(10):1232-1239; Lane et al. Nat Genet. 2017, 49(2):274-281; Dashti et al. Nat Commun. 2019, 10(1): 1100; Jones et al. PLoS Genet. 2016, 12(8):e1006125; Jones et al. Nat Commun. 2019, 10(1):1585). However, the SNPs themselves impact habitual sleep duration only marginally (*e.g.,* only a few minutes longer sleep per day). Thus, while GWAS investigations have pointed to a role of *PAX8* in sleep duration, specific sequence variability in the *PAX8* gene is unlikely to be a major contributing factor in pathological hypersomnia.

While GWAS help identify potential genes linked to phenotypic traits, additional methods are needed to help diagnose and treat patients and examine specific identified genes that relate to the pathophysiology of human disease.

### SUMMARY OF THE INVENTION

According to an aspect of the present invention there is provided a method as specified in claim 1.

In some embodiments, the one or more human genomic positions align to 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, or each of positions 113992930 of chromosome 2, 113993052 of chromosome 2, 113993142 of chromosome 2, 113993304 of chromosome 2, 113993313 of chromosome 2, 113994035 of chromosome 2, 113994578 of chromosome 2, 113995370 of chromosome 2, 113995456 of chromosome 2, 124985406 of chromosome 9, 124985756 of chromosome 9, 124987896 of chromosome 9, 124988720 of chromosome 9, 124989052 of chromosome 9, 124989241 of chromosome 9, 124989294 of chromosome 9, 124989337 of chromosome 9, 124989408 of chromosome 9, 124989550 of chromosome 9, 124989839 of chromosome 9, 46391392 of chromosome 4, 46391436 of chromosome 4, 46391448 of chromosome 4, 46391476 of chromosome 4, 46391524 of chromosome 4, 46391532 of chromosome 4, and 46391694 of chromosome 4, of UCSC hg19 human reference genome.

In some embodiments, the one or more human genomic positions align to one or more, two or more, three or more, four or more, five or more, six or more, seven or more, eight or more, or each of positions 113992930 of chromosome 2, 113993052 of chromosome 2, 113993142 of chromosome 2, 113993304 of chromosome 2, 113993313 of chromosome 2, 113994035 of chromosome 2, 113994578 of chromosome 2, 113995370 of chromosome 2, and 113995456 of chromosome 2, of UCSC hg19 human reference genome. In some embodiments, the degree of methylation at the one or more human genomic positions indicates total sleep time in the subject.

In some embodiments, the one or more human genomic positions align to seven or more, or each of positions 113993142 of chromosome 2, 113993304 of chromosome 2, 113993313 of chromosome 2, 113994035 of chromosome 2, 113994578 of chromosome 2, 113995370 of chromosome 2, and 113995456 of chromosome 2, of UCSC hg19 human reference genome.

In some embodiments, the one or more human genomic positions align to each of positions 124985406 of chromosome 9, 124985756 of chromosome 9, 124987896 of chromosome 9, 124988720 of chromosome 9, 124989052 of chromosome 9, 124989241 of chromosome 9, 124989294 of chromosome 9, 124989337 of chromosome 9, 124989408 of chromosome 9, 124989550 of chromosome 9, 124989839 of chromosome 9, 46391392 of chromosome 4, 46391436 of chromosome 4, 46391448 of chromosome 4, 46391476 of chromosome 4, 46391524 of chromosome 4, 46391532 of chromosome 4, and 46391694 of chromosome 4, of UCSC hg19 human reference genome.

In some embodiments, the one or more human genomic positions align to six or more, seven or more, eight or more, nine or more, ten or more, or each of positions 124985406 of chromosome 9, 124985756 of chromosome 9, 124987896 of chromosome 9, 124988720 of chromosome 9, 124989052 of chromosome 9, 124989241 of chromosome 9, 124989294 of chromosome 9, 124989337 of chromosome 9, 124989408 of chromosome 9, 124989550 of chromosome 9, and 124989839 of chromosome 9,46391392 of chromosome 4, 46391436 of chromosome 4, 46391448 of chromosome 4, 46391476 of chromosome 4, 46391524 of chromosome 4, 46391532 of chromosome 4, and 46391694 of chromosome 4, of UCSC hg19 human reference genome.

In some embodiments, the one or more human genomic positions align to one or more of positions 124985406 of chromosome 9, 124985756 of chromosome 9, 124987896 of chromosome 9, 124988720 of chromosome 9, 124989052 of chromosome 9, 124989241 of chromosome 9, 124989294 of chromosome 9, 124989337 of chromosome 9, 124989408 of chromosome 9, 124989550 of chromosome 9, 124989839 of chromosome 9, of UCSC hg19 human reference genome.

In some embodiments, the one or more human genomic positions align to one or more of positions 46391392 of chromosome 4, 46391436 of chromosome 4, 46391448 of chromosome 4, 46391476 of chromosome 4, 46391524 of chromosome 4, 46391532 of chromosome 4, and 46391694 of chromosome 4, of UCSC hg19 human reference genome.

In some embodiments, the primers consist of fewer than 60 total primers, fewer than 54 total primers, fewer than 48 total primers, fewer than 42 total primers, fewer than 36 total primers, fewer than 30 total primers, fewer than 24 total primers, fewer than 20 total primers, fewer than 18 total primers, fewer than 16 total primers, fewer than 14 total primers, fewer than 12 total primers, fewer than 10 total primers, fewer than 8 total primers, fewer than 6 total primers, fewer than 4 total primers, or fewer than 2 total primers.

In some embodiments, the primers comprise one or more pairs of primers. In some embodiments, each pair of primers amplifies a DNA fragment of 100-400 bp, 150-350 bp, or 200-300 bp in length.

In some embodiments, the one or more pairs of primers consists of fewer than 30 pairs of primers, fewer than 25 pairs of primers, fewer than 20 pairs of primers, fewer than 18 pairs of primers, fewer than 16 pairs of primers, fewer than 14 pairs of primers, fewer than 12 pairs of primers, fewer than 10 pairs of primers, fewer than 9 pairs of primers, fewer than 8 pairs of primers, fewer than 7 pairs of primers, fewer than 6 pairs of primers, fewer than 5 pairs of primers, fewer than 4 pairs of primers, fewer than 3 pairs of primers, fewer than 2 pairs of primers, or 1 pair of primers.

In some embodiments, the biofluid is selected from the group consisting of saliva, blood, cerebrospinal fluid, sweat, tears, semen, and urine. In some embodiments, the biofluid is blood or saliva.

In some embodiments, the subject is a human.

In some embodiments, the biofluid sample is from a healthy subject.

In some embodiments, the biofluid sample is from a subject with a mean sleep latency as measured by a multiple sleep latency test of less than 10 minutes.

In some embodiments, the biofluid sample is from a subject with a pupillary unrest index as measured by infrared pupillometry of at least 9.8.

In some embodiments, the biofluid sample is from a subject demonstrating lapses in a psychomotor vigilance test of at least 4.8.

In some embodiments, the biofluid sample is from a subject previously diagnosed with diabetes, asthma, or hypertension.

The degree of methylation for any one the one or more human genomic position in any one of the aforementioned embodiments can be as shown in Tables 1 and 2.

Where the degree of methylation at the one or more human genomic positions indicates hypersomnolence in the subject, a wake promoting medication may be administered to the subject. The wake promoting medication may be an amphetamine or modafinil. Where the degree of methylation at the one or more human genomic positions indicates hypersomnolence in the subject, an oxybate or derivative thereof can be administered to the subject. Where the degree of methylation at the one or more human genomic positions indicates hypersomnolence in the subject, the subject may undergo sleep extension therapy.

Also disclosed is a method of amplification, such as a method of amplifying a differentially methylated position (DMP).The method may comprise providing a reaction mixture comprising bisulfite modified target DNA from a biofluid sample from a subject and primers specific to one or more human genomic positions, heating the reaction mixture to a first predetermined temperature for a first predetermined time, cooling the reaction mixture to a second predetermined temperature for a second predetermined time under conditions to allow the primers to hybridize with complementary sequences on the bisulfite modified target DNA, and repeating the heating and the cooling, wherein an amplified target DNA sample is formed.

The one or more human genomic positions can comprise any of the one or more human genomic positions as described above in any combination.

The primers may consist of fewer than 60 total primers, fewer than 54 total primers, fewer than 48 total primers, fewer than 42 total primers, fewer than 36 total primers, fewer than 30 total primers, fewer than 24 total primers, fewer than 20 total primers, fewer than 18 total primers, fewer than 16 total primers, fewer than 14 total primers, fewer than 12 total primers, fewer than 10 total primers, fewer than 8 total primers, fewer than 6 total primers, fewer than 4 total primers, or fewer than 2 total primers.

The primers may comprise one or more pairs of primers. Each pair of primers may amplify a DNA fragment of 100-400 bp, 150-350 bp, or 200-300 bp in length.

The one or more pairs of primers may consist of fewer than 30 pairs of primers, fewer than 25 pairs of primers, fewer than 20 pairs of primers, fewer than 18 pairs of primers, fewer than 16 pairs of primers, fewer than 14 pairs of primers, fewer than 12 pairs of primers, fewer than 10 pairs of primers, fewer than 9 pairs of primers, fewer than 8 pairs of primers, fewer than 7 pairs of primers, fewer than 6 pairs of primers, fewer than 5 pairs of primers, fewer than 4 pairs of primers, fewer than 3 pairs of primers, fewer than 2 pairs of primers, or 1 pair of primers.

The biofluid may be selected from the group consisting of saliva, blood, cerebrospinal fluid, sweat, tears, semen, and urine. The biofluid may be blood or saliva and the subject may be a human.

The reaction mixture additionally may comprise a polymerase and free nucleotides comprising adenine, thymine, cytosine, and guanine. The reaction mixture may additionally comprise a reaction buffer and MgCl₂. At least one of the primers may be biotinylated.

Also disclosed is a biomarker panel. The biomarker panel may comprise a probe, primers, or a combination thereof specific to one or more human genomic positions. The one or more human genomic positions can comprise any of the one or more human genomic positions as described above in any combination.

The primers may consist of fewer than 60 total primers, fewer than 54 total primers, fewer than 48 total primers, fewer than 42 total primers, fewer than 36 total primers, fewer than 30 total primers, fewer than 24 total primers, fewer than 20 total primers, fewer than 18 total primers, fewer than 16 total primers, fewer than 14 total primers, fewer than 12 total primers, fewer than 10 total primers, fewer than 8 total primers, fewer than 6 total primers, fewer than 4 total primers, or fewer than 2 total primers.

The primers may comprise one or more pairs of primers. Each pair of primers may amplify a DNA fragment of 100-400 bp, 150-350 bp, or 200-300 bp in length.

The one or more pairs of primers may consist of fewer than 30 pairs of primers, fewer than 25 pairs of primers, fewer than 20 pairs of primers, fewer than 18 pairs of primers, fewer than 16 pairs of primers, fewer than 14 pairs of primers, fewer than 12 pairs of primers, fewer than 10 pairs of primers, fewer than 9 pairs of primers, fewer than 8 pairs of primers, fewer than 7 pairs of primers, fewer than 6 pairs of primers, fewer than 5 pairs of primers, fewer than 4 pairs of primers, fewer than 3 pairs of primers, fewer than 2 pairs of primers, or 1 pair of primers.

The biomarker panel may comprise a probe specific to each of the one or more human genomic positions.

The biomarker panel may comprise probes and the probes consist of fewer than 28 probes.

Either the probes or the primers may be arrayed on a substrate.

The substrate may be selected from the group consisting of a chip, a bead, a plate, a microfluidic device, or a multiwall plate.

The biomarker panel may comprise: a probe specific to each of the one or more human genomic positions; primers specific to each of the one or more human genomic positions; and a carrier. The carrier may be a substrate. The substrate may be selected from the group consisting of a chip, a bead, a plate, a microfluidic device, or a multiwall plate. The probes may be arrayed on the substrate. The carrier may be a buffered solution.

### BRIEF DESCRIPTION OF THE DRAWINGS

The patent or patent application file contains at least one drawing in color. Copies of this patent or patent application publication with color drawings will be provided by the Office upon request and payment of the necessary fee.

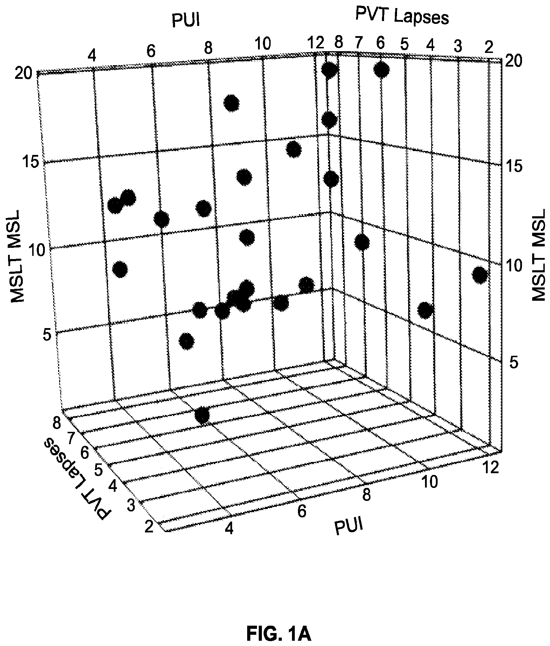

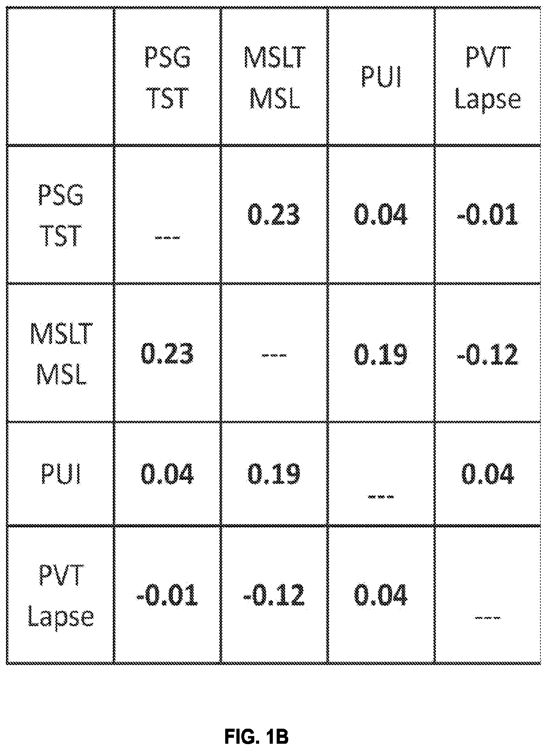

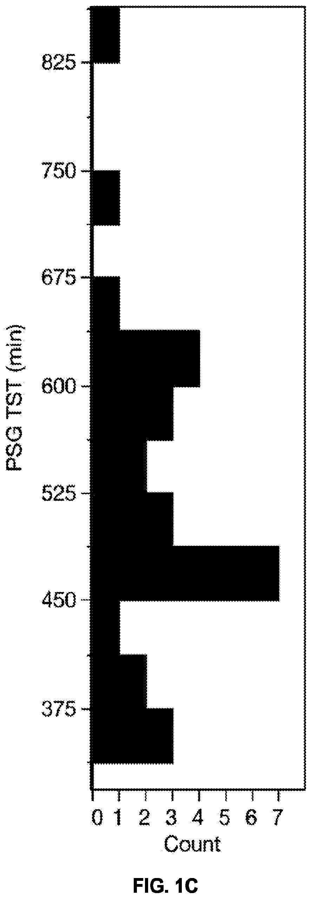

FIGS. 1A-1C show quantification of hypersomnolence variables. A) 3D-scatterplot of, MSLT mean sleep latency (MSL), pupillary unrest index (PUI), and PVT lapses in full data sample (N=28). B) Correlation (r) matrix for all hypersomnolence variables (p>0.2 for all associations). C) Histogram of ad libitum PSG TST (mean 8.7±1.9 hours; range 5.8 to 13.9 hours).

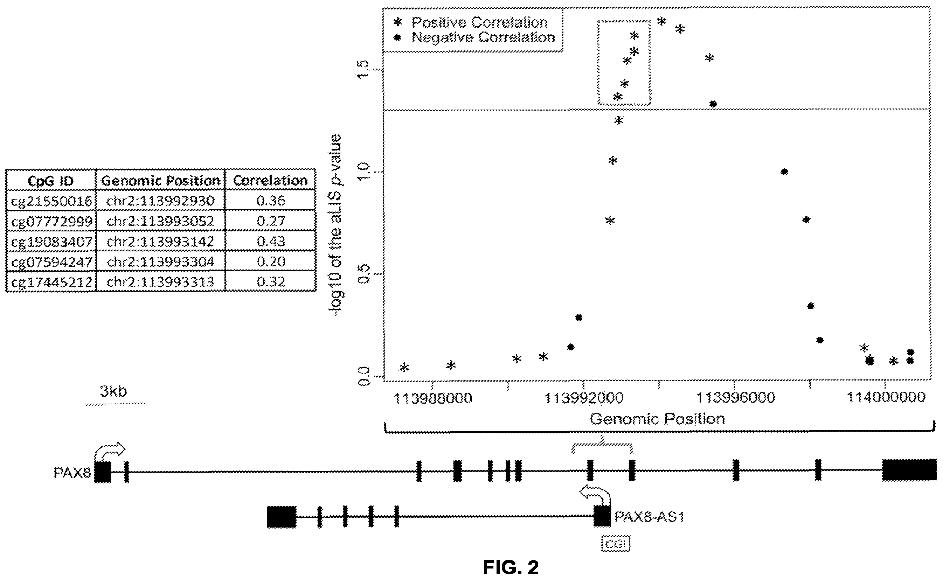

FIG. 2 shows relative positions of PSG-associated differentially methylated positions at the *PAX8* and *PAX-AS1* loci. Schematic of *PAX8* and its antisense gene *PAX8-AS1.* The relative positions of probes measuring methylation levels of CpG sites annotated to *PAX8* and *PAX8-AS1* with their genomic 5'-3'positions are provided (inset panel; x-axis) vs. the -log10 of the adjusted local index of significance (aLIS) *P*-value (y-axis). All probes were tested for positive correlations with PSG (red dots) and negative PSG (black dots) sleep duration. The level of aLIS *P*-values <0.05 (black line) is indicated. Dotted blue box denotes cluster of 5 CpG sites near *PAX8-AS1* promoter. The inset table provides the Illumina provided CpG ID number (CpG ID), the CpG chromosome (chr) number and genomic position, and the correlation R matrix between DNA methylation levels and total sleep duration for these 5 CpG sites.

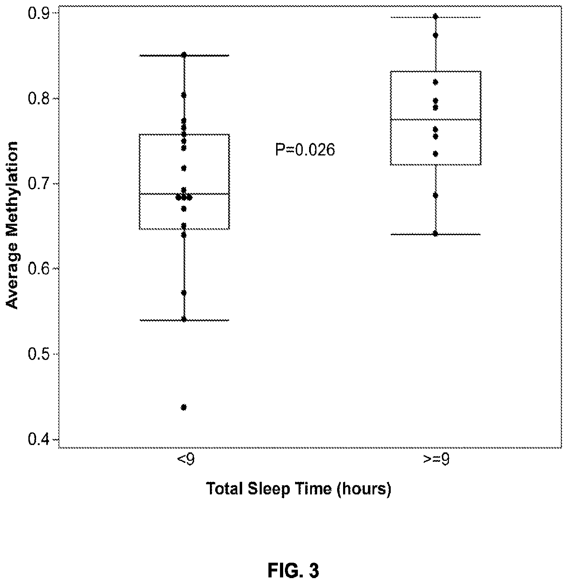

FIG. 3 shows average percent methylation of 5 DMPs in PAX8-AS1 promoter stratified by total sleep time above and below 9 hours. Average methylation was 8.6% greater in those with excessive sleep duration at this threshold (77.4% vs. 68.8%; P-value = 0.026).

### DETAILED DESCRIPTION OF THE INVENTION

Subjects with unexplained hypersomnolence have differentially methylated genes that are implicated in idiopathic hypersomnia and hypersomnolence associated conditions.

The present disclosure describes biofluid based assays for the diagnosis, prognosis, or therapeutic treatment recommendation of hypersomnolence, idiopathic hypersomnia, and acute or chronic long sleep time in a subject. The present disclosure describes differentially methylated positions (DMPs) associated with the paired box 8 (PAX8) gene and the paired box 8 anti-sense gene (PAX8-AS1) that are characteristic of hypersomnolence, idiopathic hypersomnia, and acute or chronic long sleep time in a subject. Also described are DMPs associated with LIM/homeobox protein 6 (LHX6) and gamma-aminobutyric acid receptor subunit alpha-2 (GABRA2). These characteristic biomarkers may be used to assay methylation in DNA isolated from a biofluid sample from a subject. These characteristic biomarkers may be used in the development of a screening panel, a resequencing panel, or a diagnostic kit for the processing of DNA isolated from a biofluid sample from a subject.

As used herein, "hypersomnolence" refers to excessive daytime sleepiness often accompanied by prolonged sleep duration. Biological and physiological causes of hypersomnolence are not universally known, many subjects may receive an ambiguous diagnosis despite comprehensive evaluation. Hypersomnolence is associated with a number of clinical phenotypes including, but not limited to reduced mean sleep latency (MSL), increased total sleep time (TST), increased pupillary unrest index (PUI), and increased lapses on a psychomotor vigilance test (PVT). Methods for measuring each of the hypersomnolence-associated phenotypes are known in the art.

In some embodiments, the hypersomnolence-associated phenotype is reduced MSL. MSL may be measured using a multiple sleep latency test in which quantifies sleep propensity during repeated nap opportunities and the presence of sleep onset rapid eye movement (REM) periods. In some embodiments, MSL measurements associated with hypersomnolence are less than 5 minutes for pathogenic sleepiness, and less than 8-10 minutes for moderate sleepiness.

In some embodiments, the hypersomnolence-associated phenotype is increased TST. TST may be measured using ad libitum polysomnography. In some embodiments, TST associated with hypersomnolence is greater than 9 hours. In more severe forms it is greater than 10 or 11 hours.

In some embodiments, the hypersomnolence-associated phenotype is increased PUI. PUI may be measured using infrared pupillometry. In some embodiments, PUI associated with hypersomnolence is at least 9.8 (unitless).

In some embodiments, the hypersomnolence-associated phenotype is lapses in the PVT. PVT is a well-validated measure used in sleep research to quantify the ability to sustain attention and response in a timely manner to salient signals. A PVT lapse is generally characterized as a failure to respond to the given stimuli within a predetermined time (typically 500 msec). In some embodiments, PVT lapses associated with hypersomnolence is at least 4.8 lapses.

As used herein, "idiopathic hypersomnia" refers to excessive daytime sleepiness even after a full or prolonged night's sleep. A subject with idiopathic hypersomnia may sleep normally or for long periods during the night but is still excessively sleepy during the day, which may cause dangerous situations if sleepiness is brought on during driving, working, or other activities. Napping generally does not relieve the sleepiness of an individual with idiopathic hypersomnia.

As used herein, "long sleep time" refers to acute or chronic periods of sleep by an individual that are longer than about 9 hours for an adult human, about 10 hours for a teenaged human, or about 12 hours for a young child.

Described herein are differentially methylated positions associated with the PAX8 and PAX8-AS1 genes that are characteristic of hypersomnolence, idiopathic hypersomnia, and long sleep time in a subject. Also described herein are differentially methylated positions associated with LHX6 that are characteristic of mean sleep latency and GABRA2 that are characteristic of pupillary unrest index.

As used herein, "differentially methylated positions" or "DMP" refers to CpG dinucleotide positions with a significant increase (hypermethylation), a significant decrease (hypomethylation) in methylation (e.g., composite of 5-methylcytosine + 5-hydroxymethylation [5mC + 5hmC]) relative to hypersomnolence clinical phenotypes, or a significant change in methylation that correlates to a hypersomnolence clinical phenotype. In some embodiments, the DMP corresponds to a position with a change in methylation that correlates to a hypersomnolence clinical phenotype as outlined in Table 1 and Table 2.

**Table 1: Differentially methylated positions within PAX8 and PAX8-AS1 associated with total sleep time.**

| **CpG ID** | **Chromosome** | **Genomic position** | **DNA strand¹** | **Relation to a CpG Island²** | **UCSC RefGene Name** | **aLIS *P*-value³** | **Correlation⁴** | **DMP Status** | **DNAm cutoff** |
|---|---|---|---|---|---|---|---|---|---|
| cg21550016 | chr2 | 113992930 | + | N_Shore | PAX8;LOC440839;LOC654433 | 0.04313874 | 0.3592121 | Hyper | >0.7 |
| cg07772999 | chr2 | 113993052 | + | N_Shore | PAX8;LOC440839;LOC654433 | 0.03748023 | 0.2653631 | Hyper | >0.7 |
| cg19083407 | chr2 | 113993142 | - | N_Shore | PAX8;LOC440839;LOC654433 | 0.02880542 | 0.4265851 | Hyper | >0.7 |
| cg07594247 | chr2 | 113993304 | - | Island | PAX8;LOC440839;LOC654433 | 0.02598576 | 0.1988143 | Hyper | >0.7 |
| cg17445212 | chr2 | 113993313 | - | Island | PAX8;LOC440839;LOC654433 | 0.02179952 | 0.3193101 | Hyper | >0.7 |
| cg21610815 | chr2 | 113994035 | + | Island | PAX8;LOC654433;LOC440839 | 0.01861881 | 0.1549738 | Hyper | >0.8 |
| cg00422909 | chr2 | 113994578 | - | S_Shore | PAX8-AS1;PAX8 | 0.02050616 | 0.3243021 | Hyper | >0.6 |
| cg13020245 | chr2 | 113995370 | + | S_Shore | PAX8-AS1;PAX8 | 0.02841775 | 0.01047076 | Hyper | >0.8 |
| cg04345118 | chr2 | 113995456 | + | S Shore | PAX8;LOC654433;LOC440839 | 0.04720507 | -0.0790697 | Hypo | <0.7 |

| | | | | | | | | | |
|---|---|---|---|---|---|---|---|---|---|
| '(+) sense and (-) antisense DNA strands ²CpG island relationships are described in detail above ³The R package NHMMfdr was used to detect the adjusted local index of significance (aLIS), an extension of adjusted P-values, for each probe by first converting all P-values to z-scores, followed by employing a Hidden Markov Model to determine the "aLIS P-value." ⁴Correlation R matrix between DNA methylation levels and hypersomnolence variables | | | | | | | | | |

**Table 2: Differentially methylated positions within other DMP-associated genes.**

| **CpG ID** | **Chromosome** | **Genomic position** | **DNA strand¹** | **Relation to a CpG Island²** | **UCSC RefGene Name** | **aLIS *P*-value³** | **Correlation⁴** | **DMP Status** | **Associated Phenotype** | **DNAm cutoff** |
|---|---|---|---|---|---|---|---|---|---|---|
| cg10264903 | chr9 | 124985406 | - | N_Shelf | LHX6 | 0.04040517 | 0.2371739 | Hyper | MSLT | <0.15 |
| cg02825412 | chr9 | 124985756 | - | N_Shore | LHX6 | 0.02633677 | 0.0431312 | Hyper | MSLT | <0.68 |
| cg09723488 | chr9 | 124987896 | + | Island | LHX6 | 0.02187027 | -0.2624312 | Hypo | MSLT | >0.7 |
| cg04282082 | chr9 | 124988720 | - | Island | LHX6 | 0.01565797 | -0.2763055 | Hypo | MSLT | >0.65 |
| cg11479503 | chr9 | 124989052 | + | Island | LHX6 | 0.0181288 | -0.186529 | Hypo | MSLT | >0.7 |
| cg21213617 | chr9 | 124989241 | + | Island | LHX6 | 0.02273288 | -0.1609523 | Hypo | MSLT | >0.7 |
| cg21469772 | chr9 | 124989294 | - | Island | LHX6 | 0.02100529 | -0.2001834 | Hypo | MSLT | >0.7 |
| cg13571460 | chr9 | 124989337 | + | Island | LHX6 | 0.02199509 | -0.1841574 | Hypo | MSLT | >0.65 |
| cg05136264 | chr9 | 124989408 | - | Island | LHX6 | 0.02387765 | -0.1936458 | Hypo | MSLT | >0.7 |
| cg05037505 | chr9 | 124989550 | - | Island | LHX6 | 0.02907199 | -0.2365778 | Hypo | MSLT | >0.6 |
| cg15124400 | chr9 | 124989839 | + | Island | LHX6 | 0.03946631 | -0.2126105 | Hypo | MSLT | >0.7 |
| cg25145765 | chr4 | 46391392 | - | N_Shore | GABRA2 | 0.03552697 | -0.1922142 | Hypo | PUI | <0.03 |
| cg02620694 | chr4 | 46391436 | + | N_Shore | GABRA2 | 0.01720495 | -0.3900152 | Hypo | PUI | <0.03 |
| cg14778074 | chr4 | 46391448 | + | N_Shore | GABRA2 | 0.01414029 | -0.2889577 | Hypo | PUI | <0.09 |
| cg02633148 | chr4 | 46391476 | + | N_Shore | GABRA2 | 0.01611223 | 0.1642397 | Hyper | PUI | >0.06 |
| cg08206959 | chr4 | 46391524 | + | N_Shore | GABRA2 | 0.01240927 | -0.2945528 | Hypo | PUI | <0.05 |
| cg14411873 | chr4 | 46391532 | + | N_Shore | GABRA2 | 0.01317009 | -0.4174173 | Hypo | PUI | <0.06 |
| cg12205729 | chr4 | 46391694 | + | N_Shore | GABRA2 | 0.02400582 | -0.3520862 | Hypo | PUI | <0.02 |

| | | | | | | | | | | |
|---|---|---|---|---|---|---|---|---|---|---|
| ¹(+) sense and (-) antisense DNA strands ²CpG island relationships are described in detail above ³The R package NHMMfdr was used to detect the adjusted local index of significance (aLIS), an extension of adjusted P-values, for each probe by first converting all P-values to z-scores, followed by employing a Hidden Markov Model to determine the "aLIS P-value." ⁴Correlation R matrix between DNA methylation levels and hypersomnolence variables | | | | | | | | | | |

As used herein, "significant change" refers to a change (i.e., increase or a decrease) with a statistical signification of aLIS P-value < 0.05 when compared to hypersomnolence clinical phenotype.

As used herein, "significant increase" refers to an increase with a statistical signification of aLIS P-value < 0.05 when compared to hypersomnolence clinical phenotype.

As used herein, "significant decrease" refers to a decrease with a statistical significance of aLIS P-value < 0.05 when compared to hypersomnolence clinical phenotype.

As used herein, "differentially methylated position-associated genes" or "DMP-associated genes" refers to the genes in which the DMPs are located or the genes with which the DMPs are most closely associated. In some embodiments, the DMP may be in the coding region of the DMP-associated gene. In some embodiments, the DMP may be in the promoter region of the DMP-associated gene. In some embodiments, the DMP-associated genes are PAX8, PAX8-AS1, LHX6, and GABRA2.

As used herein, "CpG island" refers to short CpG-rich areas defined as >0.5 kb stretches of DNA with a G+C content greater than or equal to 55% and an observed: expected frequency of at least 0.6. In general, CpG are located in the promoter region and 5' to the transcription start site, although a CpG island may occur anywhere in a gene. Many terms of art are associated with characterization of CpG sites and differential methylation relative to a particular gene. A CpG shore refers to a region with lower CpG density than a CpG island that lies within the 2 kb up- and downstream of a CpG island. A CpG shelf refer to the 2 kb outside of a CpG shore. A CpG canyon refers to regions of low methylation more than 3.5 kb distant from CpG islands and CpG shores that frequently contain transcription factors. A CpG ocean refers to regions with low methylation and not characterized as either a CpG island, CpG shore, CpG shelf, or CpG canyon. A gene body refers to the entire gene from transcription start site to the end of the transcript. A gene desert refers to regions with very few, if any, genes in a 500 kb region. See, for example, Asmar et al. (DNA methylation and hydroxymethylation in cancer, Epigenetic Cancer Therapy, 2015, p. 9-30).

DMP biomarker candidates associated with PAX8 and PAX8-AS1 show significant (aLIS P-value < 0.05) association between degree of methylation in target regions and total sleep time measured with ad libitum polysomnography when DNA samples from subjects with hypersomnolence are analyzed. DMP biomarkers associated with PAX8 and PAX8-AS1 are recited in Table 1.

In some embodiments, the CpG sites of interest within an about 400 base pair region (SEQ ID NO:1) located in the coding region of the PAX8 gene and near the promoter of the PAX8-AS1 gene. All DMPs in this about 400 base pair region are hypermethylated (*i.e.*, increasing methylation is associated with longer sleep time). In some embodiments, the CpG sites of interest are within the region defined by SEQ ID NO:2.
SEQ ID NO:2 (CpG DMPs indicated in **BOLD**, the -400 bp region of SEQ ID NO:1 is in *italics*):

CGCCGCCATAGCTGCATGGCCCCGGGACCTCCCTGTCGTACCTGAGAGGAGGGCCTGG
CCCGTGAACTGCCCGTACACGGAGGCAGCATGGGGAAAGGCATTGAAGGGCGGGACCC
CGGAGCCGACTTGCTGCAGATCCAAAAAGGCGGAGCTAGATAAAGAGGAAGGGGTGGAG
CTAGAACTGGACACCTCGGGGGTTTCCTGCTTTATGGCGAAGGGTGAGTGAGGATCTGC
CGGAGGGAGGGAGACAACAAGGAGAGAGGGGTGTGAGATGGCGGGGAGGGAACACGC
ACAAGCCAAGCCGTGGGATGTGGAGGGTGCGGGCGGGGCGCGGGGCAGGCTCAGCTG
CCCTCAGAGTCTGGGCTGGGGAGAGCCGGGGCCCACGAGGCGTGGCAAGGCGGGGA
GAGAGAAGCTAGACCTCCCTGCTGCGCTTCTGCAGCGAAAATGGAGAGACCCGGAA
GGCGTGTGTGCGCCCGCCTCTCCCACAGGAGGGAGCGCGGAGACCCGGGAGCGGCC
TAGGACCGGAGGCGCGACCCCTCGGCCCACCTTGAGGCCCGGCCTAGGACCGGAGG
CGCGACCCCTGGGCCCACCTTGAGGCCCGGCCTAGGACTGGAGGCGCGACCCCTCG
GCCCACCTTGCGGGAGCCGCCTAGGACCGGAGGCGCGACCCCTCGGCCCAGCTTGA
GGCCCGGCCTAGGACCGGAGGCGCGACCCCTCGGCCCACCTTGCGGGAGCCGCCTA
GGACCGGAGGCGCGACCCCTCGGCCCACCTTGAGGCCCGGCCTAGGACCGGAGGCG
CGACCCCTGGGCCCACCTTGAGGCCCGGCCTAGGACTGGAGGCGCGACCCCTCGGC
CCACCTTACGGGAGCCGCCTAGGGCCGGAGGCGCGACCCCTCGGCCCAGCTTGAGG
CCCGGCCTAGGACCGGAGGCGCGACCCCTCGGCCCACCTTGAGGCCCGGCCTAGGA
CTGGAGGCGCGACCCCTCGGCCCACCTTGAGGCCCGGCCTAGGACCGGAGGCGCGA
CCCCTGGGCCCACCTTGAGGCCCGGCCTAGGACTGGAGGCGCGACCCCTCGGCCCA
CCTTGAGGCCCGGCCTAGGACCGGAGGCGCGACCCCTGGGCCCACCTGGCGGCCCG
GCCGGCACAGCCCGCCTCTCCTCTCCAGGCCAGGGCCCCACACCTTCCGCCTGACAG
CCAGCCAAGCTCTTCAGTCCCCCGCCCTCCACCTGCCAGGGAGGCTCCGGGCGTTGT
ACCTGCCACCACGGGGTAGGTCTGGTGAGTCGAGAGGTTGCGCCCCAGTGGCGTGT
TGGAAGGGGTCAGGGTGGCCTTCCCGTCGTCCAGGGTGCTGTTGAGCAAGGGCAGC
GGGTAGAGGCCCTGGGGAGCAAAGAGAAGTCAGCGCACAGAGACGATCCAACAAG
CCGACCTTCCTGCAGACCCTGAGCCTGTGTCTTAGGACTTGGCAGCCCTCATCTCCC
CAGGAGAGGTCCTCATCGCCCCCTTCTTCCTTTACGTTCTCAACTCCACTCGGCACG
TTTCGTTCATGCTCATTGTCCTCCCGCATCCCTGGAATTCAAGGCCAGCTGGTAGCA
TGGGTGCCCAGACGTGGGATAGAGAGTCTCAGCCAGAGGACCACACCCTGGTTGGA
TGGCTGGTCGGCTTCCTGGTTTCACCACACTGTTGGGACCCACAGAATGCAGGACG

GTGCCAAGGACTGGAGCAGTCTCCAACCGGACTGTTCAGGGTCCTTGCATCTGTTAA
TTTATTCTTCCCCAGATAGGAAACCCAGGCACAAAGGTTTAAGAAACTTGTCCCACT
TCCAGATGGGCCAAAGATAGGATTCAGATTCGAGGGATCAGAATGTGTCTGCTCTC
AACCACTGCCCTAAACTGGGTGACATAAGGACCACTTCTTACATTTGCAAAGAGCTT
ATATCAGAGCTTTGCCTCTGTTGTCCTAGTAGAGTCCCCCAGCAGCCCAGTGACATG
GGTGAGTAATATTATGTCTGTGTTTTATATGTGGAGCCTGGAACTCAGGTAAATGGC
CCAATTTTGTATAGTGAAACAGACTAAACCCGGGATTTTTCGGCTGTGGACCCAGGT
GCTTTACCCACCACGTGGATTTCTTCTTTTCTCTCTCCACATCCAAACAAATAGAAGT
GTGCAAGTTCGGCTTGAAAATGGTATTGAGAGTTGTTTTTGTTGGAAACACTCTTTG
TCAAGTACCTGGGGGTCTTCATCAGTAAGACCTCATTTTAGATGCCTCCTGGCCTCC
CCTTCCCAGGAGCACAGCTATGACCTTAGGTACTCCTTCCGAAAAGAACTTGTTTAA
CTAAAGGTAAGTGTACCTCATCCTCACCATGGCCTCCTTCCACTGGGGAAGCAGATA
GCGCAGAAAAAAGAACACACCCATTCCCCACATACCTTCACACTCGTCACATACCT
GCTACGTGAGATGTGCAAAGCTGAATTCAGGGAATGCTCAGTAGTTACATAACAGT
GCCACTAAAGGCAATTGTTTTCAGTGATTTCCATCG

DMP biomarker candidates associated with LHX6 show significant (aLIS P-value < 0.05) association between degree of methylation in target regions and mean sleep latency measured by multiple sleep latency test when DNA samples from subjects with hypersomnolence are analyzed. DMP biomarkers associated with LHX6 are recited in Table 2.
SEQ ID NO:3 (CpG DMPs indicated in **BOLD**, the top candidate region of SEQ ID NO:4 is in *italics*)

CGATCGGCTTTTTCTTTCCCTTTTTGTGGGTTTTATTATTTTTGTTACAGGTGGGGCT
GCCAGGGATGGGTTATGCCTGCAGGGGGAGAAATGATCAGAAGAGCCACAATTCTA
CCCTCAACCCAGGGAATGTGAGCTTTTACTGCCTCCATTTTCTTAACCAGAAATCTG
GTTTAGTTTTGCTTTCAGGGCTCTTGTCATTGGAAAAAGATTAGTGCTCAGAACCTG
CAGCCCAGAAATGGCCCAGTCCCATCCCTCCAGGCAGGCTCTGCTCCTCAAGCTGC
GTGGGGAAATGCCATCATGGGCTTGTGCCCAGCATCCACCCTGCCAATGATGACAA
CAGTGATAATCGAGGCATTCATACCAACCACCATTTAGGGAGCCTTTATAGAGACT
TCCCCCCAACAGTGGCCTCTTTGGCAGCTCTGGGAGACAGCCTTGGAAAGGGCGAA
CTAAGGGCCGAGAGAAGCAGCAACTTGCGAAGGTCACTACTGGCCAGACTGGGCCC
AGTCTCCTGCCTCTTCCCACAGTGGCTGCACACCCTACTCTCTACCCCATTGCAGTA
GGAAGAGAACTCCAGAATAAGGGGAGGCTCTGAACAGTGGAGTCAGGATCCGTGT
TTTGACTAGTTTCAAGGTCTAGTAAAGCACAGTATCCAAGGAGGCTCAGTCCAGCA

TGGGCATGGGCTAGGGCTGGAGAAGTAGAAAAGAAATTTGGGCAAGTAGTGAATA
AGACTGCCAAATCTATTGTTAGGAGAGCCAGGAGAGGTGTCCTAGTGGTGCCTTAG
TCTATGGGAGAGACCCAGACACCATTCTGCTGGGTTCCCAAGCTGGCACCTTAAATT
TAGTAGCTGATGAATCCAAGGAGCCCCCAAGCATCATCTGGCATGTTTTTTTGTATG
TTTGTTTGTTTGTTTTTTGAGACAGAGTCTCTCTCTGTTACCCAGGCTGGAGTGTAGT
GACGTGATCTTGGCTCACCACAATGTCCGCCTCCCAGGTTCAAGTGATTCTCTTGCC
TCAGCCTCCTGAGTAGCTGGGACTACAGGCACGTGCCACCACACCTGGCTAAGTTTT
GTATTTTTAGTAGAGATGGGTTTCACCATGTTGGCCAGGATGGTCTCGATCTCCTGA
CCTTGTGATCTACCAGCCTTGGCCTCCCAAAGTGCTGCGATTATAGGTGTGAGCCAC
CACACCCGGCCCATCTGGCATGTTTTATACCAACTGGAGTCAGGCCTAGAGCAGGG
AGGTGGCTAGACAGTGACTGTAACCTCCATTTCCTGTCCTGGCCAAACCAATACTCA
GATGGGACAGTGAGAGGAGACATCACACTCTGGTTACACACCATTAACCCCTGTCT
GACTACACACACACACACACACAGCCGTCCAGATTCACAAATACGTTCTTATCACC
CCCTTCATCCCTCAGTGCCTGGGTGGAGGCCCTTAAGCAGGAATGCCCCCTCAGAG
GAGAGGGTGGGAGTGGAATGATGGGATTTCAGAATCCCCTCACCTCACCCTCTCAC
CTCCAGATCCTAGTCCAGATGTAATCCTGTCCTCACCACTTTGCGTGCACGTGCACG
CACACACACATGCACACACACAGTCTCAACATTCACATACAGACACCCACTCTTGA
CACCCACACATGAACTAAAAGACAAACGTTGGTCCATAAAGGAACACACATGCTAT
CTACATACCCCCAACCCTGCCCAGTGATGCTTTCCCCAGCACTACTGCACAGATCCC
AGACAATACAGACACACAGGCACTCCTTGCACACTGAGTCCTAGGCCACTTGGTTG
AGCTGCCCCCACCCCACCCCAGCTGCACACTCTTCTACATCCATCCCATAAAGGAAG
AGTCCGTCTCTTTAACCATGCTTCTCATGGTTTTCAGAGTACCCTTGCCCCAAATGCT
GCCAACCTCCCCCTAGACTCAAGATCTTCTCTTTCCAGATCGCTGGAGGCTCAATGC
CAGTGCACAGGAGGAGATCGCCTTCCACCATATTCCCTGACTCCCAGAGGCTCTTTC
TAGGGCAGGGGTCTGGCCAGGAAGACTGAAGCCATGACGTCTTCCTGTGCAAGGAT
AGGAAAGATGTCTGTGGGTTGGAGGGTAGTTATAGGGAAGCAGCTTTCTCCACTTTT
TGAGAAAGCACTTGGGAACAAGCATCCCTCTCAGGAAGTGATTCTCCACCATTCTCC
TCTGATCCTATAGGCCTGGTGGTCCAAGCCTCAGGCCAGTTGATGCCTCCCACTGGG
ATCAGCAGACAAGGCCATGACCCTGCAGTGACCTCGACCCCATAATCAGGTCAGGT
CCGGGCTTCATGGCTAGAAGCGAGGCCCAGGCGAGTGGCCAAGACGCGGCAGGAC
AGGAGGATGCGCCTGGCTGCTCTCGGCTGCTGGACGCGGCCTGCAGTAGGGAGAGC
GCAGCCCGGGGTAATCCCACTCAGGGCTCCGGGTGCCCAGTTCTCCCACTTCCCATC
CTGGCGGCCTAAAGGCAAGGCCTAGACCCTGGAGAGGCCAAAGTCGGGCCAAAAC
AACAGCGAAGCTGGGACTGGAACAGGCGCTGGGGGCCTGCGCATGACCCCAGGCT
CAGAATGCGACACTCCAGTGACGCCGGGGAATGAGTTGAGATGGAGCCGGAGACA

GAGGTTGGGGTTCAGGACGAGGCGCTTGGGTATGGGATCGGATCTGAGACGGTTGC
GGCGCTGGTGCTTTGGGCTGGACCAGACTCGAACCCAGAGCGGAGCGAGGGTCAGA
GCCAGGGCAAGAGTCCGAAGCCCAGCTCAGCGTCCAGCCCGCGAGAGACCCCACC
ATGAATGACGTCCGAGTAGGGTTGGAACTCGAAGCCCGAACGCACTGGAAATGCAG
CCGGACGACGGCTGGGATCCGAGTGGGTCCGGGCCAGAAATGGGGACCTCAGAGC
TCCACCGAGGCGCTCGAGGTCAGAACTGAAGCCTGAGACTTAGGCAGGACCCGAGA
CAGAGCCAGAGACGATACCGAAACCCAATGGACCGCGAGGACCGAAGGCAGATCC
GGGGCGCAAACCTGTGCAGGCACTGGCGCGCTGCCTGGAGCTCTGGGTTCGCGCCG
CTGAGCGCCGGCAGGTTGGACCAGCGCTGCGCGCCGGAAGTGCACTCGGGACGCCG
GGGTTCTGGAGGAGAGACCACACGCCGCAAAAGTGGCCTCCGAATGCGCCCGGGG
CCTGCCCTCGGCGACACTGCTGGCTCGGCGGCCGCAGCCTACCTGAAGTAGTCCATC
TTGCAGAAGATCTCCTTGTTCTTGATGTAGCAGCTGTTCTGCTGCCTCAGCGACGTG
CGACACACGGAGCACTCGAGGCACCGCACGTGCCAGATGAGGTTGTTGACCTGGGG
ACGGGGCGGGGACGGAGGTCGGCTCAGCGCGGGCCTCTTTCACTCCGGGCCCCAGC
CTTCCCGGTCTCATTTACAGTCAGAGGCGCTGAAGCTCAGAAGGTGCATGCGATTCC
CCTATTTTTCTCCCGTAATACTGAGACTCAGGGAAATGGAAATAAACTCTTCTTATT
TTTCAGACGGAACCCGGGGGCTCAGGCAGACCCTAAGTCTTGCCCAAAGCTTCGCA
GTCGGGCAGCAGTGGAGCCAGGATTTGGAAGTAAGAGGACCTGCGGTGCTTCCCTG
CAGTCTCCTGCGCTGCGTCCCACGCCCCGACAACACGCACGCAACACCTACCCCTGTCT
CACCTTGAGCAGATATCGGTCCAGGATCTCGAGGCCGCAGCTGGAGCAGATGTTCTTGC
CTGCAGACGGCACGGAGGAGGCGGCAGAGGGCGGTGAGCAGACAGATGGCGTGCTGG
GCGTACATGGGGAGGCCTGACCCTCGTCCTTGTCCAGAGCTCCTGCCAGGGCCTCGGC
GTCAGACTGAGCCTGCGGCGGGGGAGAGAAGGAGAGGAGCGCTGATCCGGGCACCCA
GAGTTGGGGCTGCCTTGGAGACAAATCTGGGGCCCCCGAGCACCAATTGACAACGAAAT
CTCCCCAGGACAATGCGCCGTAGACTGAAGGACAGGGATGGAAGGGCCTATTGGAGAGG
AAGGGACGGCCCCAACTTTTAACACGCGCATTCTGGGTGCTGGGAGCGGTTAAAGCCAA
CATTCCCTGCCGGAAAACCGAGGCTCAGAGAGGGGCGGCGCCCGCCGGGAGTCCCCC
AGAGCGTGAGCAGCAGTTCGTGGCCGGAAAACCTGGCCAGGTCCCCAGGGCCGGG
CCGCTGGAGGACTGGGCACCGCAGGCAGGGCCAAACGGACTCACCATGGCGGGCG
GCGCGGTCCCTTCAAGACAGCGGGTGGTCGCTTTGCAGCCGGACCCTGGCTGGGCC
ATCACCTGGGGGAGGGGGGGAGGGAACGCAGGCGGCGGCGGCTGCTGAACTGGCT
CCGCACAGCCTGAGCCCAGCGCCTCCCCGCG

DMP biomarker candidates associated with GABRA2 show significant (aLIS P-value < 0.05) association between degree of methylation in target regions and pupillary unrest index measured by infrared pupillometry when DNA samples from subjects with hypersomnolence are analyzed. DMP biomarkers associated with GABRA2 are recited in Table 2.
SEQ ID NO:5 (CpG DMPs are indicated in **BOLD**)

CGTTTGACAGCTGCTCTGGAGCCGAGGATCACAATAAGAGAAGGCGCGTGAGGCT
CCGGCAGCAAACACCAGCCAGGCAGGCAGCGGTAATCACAGTGAGCAAAGTTGAA
GACTATAAAAGAGCCAGGCTAACGTGCTGCCGAGCAGGTGTCCTGGATTTTATTGT
AGTTGCAAATATGCTTCTGGTGATAAGTATCGAAATTATTTTTTAAGTGCGCTGGGG
AGGAGGGAGGTGCGGGAATTGAGCCTCCTCCGTTTCCAGGGAGCCTCTGAAAGTGG
GGTGGGGGGAGATGAGGCATGCACG

Any combination of DMPs outlined in Tables 1 and 2 may be used to diagnose or give a prognosis of hypersomnolence, idiopathic hypersomnia, or long sleep time in a subject. Any combination of DMPs outlined in Tables 1 and 2 may be used in an assay to quantify methylation. Any combination of DMPs outlined in Tables 1 and 2 may be used in an assay to amplify the DMPs or DMP-associated genes for sequencing to quantify methylation.

In some embodiments, the DMPs of interest are within any one or more of SEQ ID NO:1, SEQ ID NO:2, SEQ ID NO:3, SEQ ID NO:4, SEQ ID NO:5, SEQ ID NO:6, SEQ ID NO:7, SEQ ID NO:10, SEQ ID NO:11, SEQ ID NO:18, SEQ ID NO:19, SEQ ID NO:24, SEQ ID NO:25, SEQ ID NO:34, and SEQ ID NO:35. In some embodiments, DMPs within any one or more of SEQ ID NO:1, SEQ ID NO:2, SEQ ID NO:3, SEQ ID NO:4, SEQ ID NO:5, SEQ ID NO:6, SEQ ID NO:7, SEQ ID NO:10, SEQ ID NO:11, SEQ ID NO:18, SEQ ID NO:19, SEQ ID NO:24, SEQ ID NO:25, SEQ ID NO:34, and SEQ ID NO:35 are assayed to diagnose or give a prognosis of hypersomnolence, idiopathic hypersomnia, or long sleep time in a subject.

In some embodiments, at least 1, at least 2, at least 3, at least 4, at least 5, at least 6, at least 7, at least 8, or all 9 of the DMPs of PAX8 and PAX8-AS1 outlined in Table 1 are assayed to diagnose or give a prognosis of hypersomnolence, idiopathic hypersomnia, or long sleep time in a subject. In some embodiments, at least 1 or both of the DMP-associated genes PAX8 and PAX8-AS1 are assayed to diagnose or give a prognosis of hypersomnolence, idiopathic hypersomnia, or long sleep time.

In some embodiments, at least 1, at least 2, at least 3, at least 4, at least 5, at least 6, at least 7, at least 8, at least 9, at least 10, or all 11 of the DMPs of LHX6 outlined in Table 2 are assayed. In some embodiments, at least 1, at least 2, at least 3, at least 4, at least 5, at least 6, or all 7 of the DMPs of GABRA2 outlined in Table 2 are assayed.

In some embodiments, the biomarkers described herein are used in the production of a biomarker panel for use in assaying DNA methylation. The biomarker panel includes probes or primers specific to the sequences of the DMPs or DMP-associated genes disclosed herein. In some embodiments, the biomarker panel includes probes or primers specific to the sequences of the DMP-associated genes PAX8 and PAX8-AS1. In some embodiments, the biomarker panel includes probes or primers specific to the sequences of the DMP-associated genes LHX6 and GABRA2. In some embodiments, the biomarker panel includes probes and/or primers specific to the DMPs listed in Tables 1 and 2. In some embodiments, the biomarker panel includes probes and/or primers specific to any one or more of SEQ ID NO:1, SEQ ID NO:2, SEQ ID NO:3, SEQ ID NO:4, SEQ ID NO:5, SEQ ID NO:6, SEQ ID NO:7, SEQ ID NO:10, SEQ ID NO:11, SEQ ID NO:18, SEQ ID NO:19, SEQ ID NO:24, SEQ ID NO:25, SEQ ID NO:34, and SEQ ID NO:35.

Primers specific to the DMPs or DMP-associated genes disclosed herein are between about 10 base pairs (bp) and about 40 bp and are complementary to sequences upstream and downstream of the DMP or DMP-associated gene of interest. Generally, a pair of forward and reverse primers that are designed to be complementary to the sequences flanking the DMP or DMP-associated gene are included. The size of the fragment to be amplified by the primer pair can range from less than 50 bp to greater than 10,000 bp. Primers can be designed that are complementary to a sequence less than 50 bp upstream of the DMP or more than 1,000 bp upstream depending on the sequence technology selected and the application of the biomarker panel. Therefore, it is possible to design many permutations of primer sets that are capable of amplifying a given DMP or DMP-associated gene of interest. For example, a given sample containing genomic DNA with a 500 bp DMP, a primer set can be designed to amplify i) the exact target region; or ii) a region encompassing the DMP including upstream and downstream regions. In some embodiments, the size of the fragment to be amplified by the primer pair has a length less than 1000 bp, less than 950 bp, less than 900 bp, less than 850 bp, less than 800 bp, less than 750 bp, less than 700 bp, less than 650 bp, less than 600 bp, less than 550 bp, less than 500 bp, less than 450 bp, less than 400 bp, 350 bp, less than 300 bp, less than 250 bp, less than 200 bp, less than 150 bp, less than 100 bp, less than 75 bp, or less than 500 bp. In some embodiments, the size of the fragment to be amplified by the primer pair has a length of at least 100 bp, at least 125 bp, at least 150 bp, at least 175 bp, or at least 200 bp and less than 400 bp, less than 375 bp, less than 350 bp, less than 325 bp, or less than 300 bp.

Probes specific to the DMPs or DMP-associated genes disclosed herein are between about 10 bp and about 40 bp and are commentary to sequences including or adjacent to the DMP or DMP-associated gene of interest. In some embodiments, the probe is complementary to the DMP of interest.

The disclosure includes a number of preferred primers and probes for amplification, selection, and identification of specific DMPs or DMP-associated genes. However, a skilled artisan will appreciate that the DMPs and DMP-associated genes disclosed can be amplified, selected, and identified by primers and probes other than those specific disclosed, which have been presented for purposes of illustration. We envision that the biomarker panel is compatible with a number of amplification and sequencing schemes and the scope of the claims should not be limited to the description of the embodiments contained herein.

Probes or primers for use in the biomarker panels described herein may be fused to a tag or label. Suitable tags and labels are known in the art, including but not limited to fluorescent labels (e.g., GFP, RFP, etc.), biotin, and combinations thereof. In some embodiments, the probe or primer is biotinylated and the biotinylated probe or primer bound sequence can be purified or captured with a streptavidin bound substrate.

In some embodiments, the primers or probes are covalently or non-covalently linked to a substrate. Suitable substrates for the biomarker panel include a bead, a plate, a microfluidic devise, a cuvette, a chip, a multiwell plate (e.g. 6-, 12-, 24-, 48-, 96-, 384-, or 1536-well plates).

Primers specific to the DMPs or DMP-associated genes disclosed herein can be obtained using a number of methods known in the art. In one example, the abundance of 5mC and/or 5hmC in regions of interest can be interrogated (see below for an exemplary method). Once regions of interest are identified, genomic sequences corresponding to each locus can be obtained from a reference genome and imported to a methyl primer design software package, such as the Thermo Fisher Scientific Methyl Primer Express v1.0 software, to generate high quality PCR primers pairs for methylation mapping. A second pair of primers can be designed that contains the initial primer sequence flanked by a sequencing adapter sequence (e.g., Illumina or others). Primers can be ordered through any preferred provider.

An exemplary method for interrogating the abundance of 5mC and/or 5hmC in regions TM of interest can be performed with the Methyl Primer Express Software v1.0 (Thermo Fisher TM Scientific). Methyl Primer Express can be downloaded from the Thermo Fisher Scientific Applied Biosystems website (www.thermofisher.com/us/en/home.html). The target sequence can be input into the box labeled "Insert Nucleotide Sequence." (To increase the likelihood of finding primers that will create an amplicon of -200 base pairs (bp) or larger, input a minimum sequence of ~500 base pairs. The longer the input sequence, the better chance of finding suitable primers to target the region of interest.) "Design Primers" can then be clicked. "Select Target Sequence" can then be chosen to select the specific region of interest within the input DNA sequence. (As above, the longer the target sequence selected, the more likely it is to find suitable primers.) Following selection of primers, "Next" can be pressed, and a new window will open. If finding CpG islands within the target sequence is of interest, "Find CpG Islands" can be clicked. (Check the current default parameters that define a CpG island. If the input sequence is only 500 base pairs, consider shortening the defined minimum length of the island, as the length of a CpG island will later serve to help in choosing where to design the primers. It is possible that these analyses may not yield any CpG islands. If this is the case, simply move on to the next step.) In the bottom right corner of the new window, bisulfite sequencing primer design can be selected by clicking "Design BSP Primers." (BSP = Bisulfite Sequencing Primers. MSP = Methylation-Specific Primers.) Parameters used to design the BSPs can be reviewed by clicking "Next." (It is preferred to design primers that will generate an amplicon length of 200 to 300 base pairs. Amplicons <200 base pairs will result in sequencing the adaptors, meaning it will not maximize the data from the final sequencing run. Primers are preferably between 20 to 30 base pairs in length. Annealing temperature of the PCR should have a small range (e.g., 58° to 60°C).) It is preferred to initially weight performance towards the Low Speed/High Accuracy setting (between 8 to 10). If no appropriate primers are found, the scale can be moved towards the Hi Speed/Low Accuracy; however, this is not preferred. The designed primers can be purchased through any of a number of standard primer providers. Exemplary parameters include 25 nmole DNA oligos, standard desalting, and RNase free water (unless shipped desiccated). Two to three primer pairs per target region can be designed and purchased for simultaneous testing.

Exemplary non-modified and bisulfite-modified regions of interest for PAX8/PAX8-AS1, LHX6, and GABRA2 comprising the DMPs disclosed herein and exemplary primers for amplifying the DMPs are provided below. All positions below refer to the UCSC hg19 human reference genome assembly.

### PAX8/PAX8-AS1

### PAX8/PAX8-AS1 Region of Interest 1: chr2:113,992,930-113,993,314 5'pad=250 3'pad=250 Non-Modified PAX8/PAX8-AS1Region of Interest 1 Sequence:

AGGCCAAGTTGCTACGTTACAGCACCCGGCTCCCGGCCTTAGGGTTCCTGCT
TTTCCATGGGGTCTTGGGGTGGTGCATCCCCGGCACCCCTACAGCATCCGCC
CCTTCCGAGCATGTCTTCCCCGTCACAGAGAACTTCATGTTGGCGCCTCCAAA
AGTTGCCGGAGGAATTAGGGAACAGGGTGGGGGTGGATGAGACTGAGGCCA
GAGAGGGGGCTGGCGGTCTGCCCTGAGGACCCCCGTCCCACCCGCCGCCATA
GCTGCATGGCCCCGGGACCTCCCTGTCGTACCTGAGAGGAGGGCCTGGCCCG
TGAACTGCCCGTACACGGAGGCAGCATGGGGAAAGGCATTGAAGGGCGGGA
CCCCGGAGCCGACTTGCTGCAGATCCAAAAAGGCGGAGCTAGATAAAGAGG

AAGGGGTGGAGCTAGAACTGGACACCTCGGGGGTTTCCTGCTTTATGGCGAA
GGGTGAGTGAGGATCTGCCGGAGGGAGGGAGACAACAAGGAGAGAGGGGT
GTGAGATGGCGGGGAGGGAACACGCACAAGCCAAGCCGTGGGATGTGGAGG
GTGCGGGCGGGGCGCGGGGCAGGCTCAGCTGCCCTCAGAGTCTGGGCTGGG
GAGAGCCGGGGCCCACGAGGCGTGGCAAGGCGGGGAGAGAGAAGCTAGAC
CTCCCTGCTGCGCTTCTGCAGCGAAAATGGAGAGACCCGGAAGGCGTGTGTG
CGCCCGCCTCTCCCACAGGAGGGAGCGCGGAGACCCGGGAGCGGCCTAGGA
CCGGAGGCGCGACCCCTCGGCCCACCTTGAGGCCCGGCCTAGGACCGGAGG
CGCGACCCCTGGGCCCACCTTGAGGCCCGGCCTAGGACTGGAGGCGCGACCC
CTCGGCCCACC (SEQ ID NO:6)

### Bisulfite-Modified PAX8/PAX8-AS1 Region of Interest 1 Sequence:

AGGTTAAGTTGTTACGTTATAGTATTCGGTTTTCGGTTTTAGGGTTTTTGTTTT
TTTATGGGGTTTTGGGGTGGTGTATTTTCGGTATTTTTATAGTATTCGTTTTTT
TCGAGTATGTTTTTTTCGTTATAGAGAATTTTATGTTGGCGTTTTTAAAAGTT
GTCGGAGGAATTAGGGAATAGGGTGGGGGTGGATGAGATTGAGGTTAGAGA
GGGGGTTGGCGGTTTGTTTTGAGGATTTTCGTTTTATTCGTCGTTATAGTTGTAT
GGTTTCGGGATTTTTTTGTCGTATTTGAGAGGAGGGTTTGGTTCGTGAATTGTTCG
TATACGGAGGTAGTATGGGGAAAGGTATTGAAGGGCGGGATTTCGGAGTCGATTT
GTTGTAGATTTAAAAAGGCGGAGTTAGATAAAGAGGAAGGGGTGGAGTTAGAATT
GGATATTTCGGGGGTTTTTTGTTTTATGGCGAAGGGTGAGTGAGGATTTGTCGGAG
GGAGGGAGATAATAAGGAGAGAGGGGTGTGAGATGGCGGGGAGGGAATACGTAT
AAGTTAAGTCGTGGGATGTGGAGGGTGCGGGCGGGGCGCGGGGTAGGTTTAGTT
GTTTTTAGAGTTTGGGTTGGGGAGAGTCGGGGTTTACGAGGCGTGGTAAGGCGG
GGAGAGAGAAGTTAGATTTTTTTGTTGCGTTTTTGTAGCGAAAATGGAGAGA
TTCGGAAGGCGTGTGTGCGTTCGTTTTTTTTATAGGAGGGAGCGCGGAGATT
CGGGAGCGGTTTAGGATCGGAGGCGCGATTTTTCGGTTTATTTTGAGGTTCG
GTTTAGGATCGGAGGCGCGATTTTTGGGTTTATTTTGAGGTTCGGTTTAGGAT
TGGAGGCGCGATTTTTCGGTTTATT (SEQ ID NO:7)

### Exemplary Primer Sequences for PAX8/PAX8-AS1 Region of Interest 1

### Amplification:

Forward primer: GAGGTTAGAGAGGGGGTTGG (SEQ ID NO:8)
Reverse primer: CGCTACAAAAACGCAACAAA (SEQ ID NO:9)

### PAX8/PAX8-AS1 Region of Interest 2: chr2:113993785-113995706 5'pad=250 3'pad=250 Non-Modified PAX8/PAX8-AS1 Region of Interest 2 Sequence:

CCTTACGGGAGCCGCCTAGGGCCGGAGGCGCGACCCCTCGGCCCAGCTTGAG
GCCCGGCCTAGGACCGGAGGCGCGACCCCTCGGCCCACCTTGAGGCCCGGCC
TAGGACTGGAGGCGCGACCCCTCGGCCCACCTTGAGGCCCGGCCTAGGACCG
GAGGCGCGACCCCTGGGCCCACCTTGAGGCCCGGCCTAGGACTGGAGGCGC
GACCCCTCGGCCCACCTTGAGGCCCGGCCTAGGACCGGAGGCGCGACCCCTG
GGCCCACCTGGCGGCCCGGCCGGCACAGCCCGCCTCTCCTCTCCAGGCCAGG
GCCCCACACCTTCCGCCTGACAGCCAGCCAAGCTCTTCAGTCCCCCGCCCTCC
ACCTGCCAGGGAGGCTCCGGGCGTTGTACCTGCCACCACGGGGTAGGTCTGG
TGAGTCGAGAGGTTGCGCCCCAGTGGCGTGTTGGAAGGGGTCAGGGTGGCCT
TCCCGTCGTCCAGGGTGCTGTTGAGCAAGGGCAGCGGGTAGAGGCCCTGGG
GAGCAAAGAGAAGTCAGCGCACAGAGACGATCCAACAAGCCGACCTTCCTG
CAGACCCTGAGCCTGTGTCTTAGGACTTGGCAGCCCTCATCTCCCCAGGAGA
GGTCCTCATCGCCCCCTTCTTCCTTTACGTTCTCAACTCCACTCGGCACGTTTC
GTTCATGCTCATTGTCCTCCCGCATCCCTGGAATTCAAGGCCAGCTGGTAGCA
TGGGTGCCCAGACGTGGGATAGAGAGTCTCAGCCAGAGGACCACACCCTGG
TTGGATGGCTGGTCGGCTTCCTGGTTTCACCACACTGTTGGGACCCACAGAA
TGCAGGACGGTGCCAAGGACTGGAGCAGTCTCCAACCGGACTGTTCAGGGTC
CTTGCATCTGTTAATTTATTCTTCCCCAGATAGGAAACCCAGGCACAAAGGTT
TAAGAAACTTGTCCCACTTCCAGATGGGCCAAAGATAGGATTCAGATTCGAG
GGATCAGAATGTGTCTGCTCTCAACCACTGCCCTAAACTGGGTGACATAAGG
ACCACTTCTTACATTTGCAAAGAGCTTATATCAGAGCTTTGCCTCTGTTGTCC
TAGTAGAGTCCCCCAGCAGCCCAGTGACATGGGTGAGTAATATTATGTCTGT
GTTTTATATGTGGAGCCTGGAACTCAGGTAAATGGCCCAATTTTGTATAGTG
AAACAGACTAAACCCGGGATTTTTCGGCTGTGGACCCAGGTGCTTTACCCAC
CACGTGGATTTCTTCTTTTCTCTCTCCACATCCAAACAAATAGAAGTGTGCAA
GTTCGGCTTGAAAATGGTATTGAGAGTTGTTTTTGTTGGAAACACTCTTTGTC
AAGTACCTGGGGGTCTTCATCAGTAAGACCTCATTTTAGATGCCTCCTGGCCT
CCCCTTCCCAGGAGCACAGCTATGACCTTAGGTACTCCTTCCGAAAAGAACT
TGTTTAACTAAAGGTAAGTGTACCTCATCCTCACCATGGCCTCCTTCCACTGG
GGAAGCAGATAGCGCAGAAAAAAGAACACACCCATTCCCCACATACCTTCA
CACTCGTCACATACCTGCTACGTGAGATGTGCAAAGCTGAATTCAGGGAATG
CTCAGTAGTTACATAACAGTGCCACTAAAGGCAATTGTTTTCAGTGATTTCCA
TCGAGCTGGGTTCTGCAAAGATCCACAGCACTTTCCGGTTGCATGCTGGGCA

CTTTTGGAAGCTGCAGTCAATTCTGGAGGCCACCAGGGCACCATTAGCACAT
AGCAGCAATTATTGACTAAATGGTGCTCTGGTTCCATGCCTTCCAAGGGGGC
CCACTTAGAGGCAGGGTGGAGTTGCTTAGGGCCTTTTTTTTTTTTTTTTTTTGT
AGACGGAGTTTTGCTCTTGTTGCCCAAGCTGGAGTGCAATGG (SEQ ID NO:10)

### Bisulfite-Modified PAX8/PAX8-AS1 Region of Interest 2 Sequence:

TTTTACGGGAGTCGTTTAGGGTCGGAGGCGCGATTTTTCGGTTTAGTTTGAGG
TTCGGTTTAGGATCGGAGGCGCGATTTTTCGGTTTATTTTGAGGTTCGGTTTA
GGATTGGAGGCGCGATTTTTCGGTTTATTTTGAGGTTCGGTTTAGGATCGGAG
GCGCGATTTTTGGGTTTATTTTGAGGTTCGGTTTAGGATTGGAGGCGCGATTT
TTCGGTTTATTTTGAGGTTCGGTTTAGGATCGGAGGCGCGATTTTTGGGTTTAT
TTGGCGGTTCGGTCGGTATAGTTCGTTTTTTTTTTTTAGGTTAGGGTTTTATATTTTT
CGTTTGATAGTTAGTTAAGTTTTTTAGTTTTTCGTTTTTTATTTGTTAGGGAGGTTTC
GGGCGTTGTATTTGTTATTACGGGGTAGGTTTGGTGAGTCGAGAGGTTGCGTTTTA
GTGGCGTGTTGGAAGGGGTTAGGGTGGTTTTTTCGTCGTTTAGGGTGTTGTTGAG
TAAGGGTAGCGGGTAGAGGTTTTGGGGAGTAAAGAGAAGTTAGCGTATAGAGACG
ATTTAATAAGTCGATTTTTTTGTAGATTTTGAGTTTGTGTTTTAGGATTTGGTAGTTTT
TATTTTTTTAGGAGAGGTTTTTATCGTTTTTTTTTTTTTTTACGTTTTTAATTTTATTCG
GTACGTTTCGTTTATGTTTATTGTTTTTTCGTATTTTTGGAATTTAAGGTTAGTTGGT
AGTATGGGTGTTTAGACGTGGGATAGAGAGTTTTAGTTAGAGGATTATATTTTGGT
TGGATGGTTGGTCGGTTTTTTGGTTTTATTATATTGTTGGGATTTATAGAATGTAGG
ACGGTGTTAAGGATTGGAGTAGTTTTTAATCGGATTGTTTAGGGTTTTTGTATTTGT
TAATTTATTTTTTTTTAGATAGGAAATTTAGGTATAAAGGTTTAAGAAATTTGTTTTAT
TTTTAGATGGGTTAAAGATAGGATTTAGATTCGAGGGATTAGAATGTGTTTGTTTTT
AATTATTGTTTTAAATTGGGTGATATAAGGATTATTTTTTATATTTGTAAAGAGTTTAT
ATTAGAGTTTTGTTTTTGTTGTTTTAGTAGAGTTTTTTAGTAGTTTAGTGATATGGGT
GAGTAATATTATGTTTGTGTTTTATATGTGGAGTTTGGAATTTAGGTAAATGGTTTAA
TTTTGTATAGTGAAATAGATTAAATTCGGGATTTTTCGGTTGTGGATTTAGGTGTTT
TATTTATTACGTGGATTTTTTTTTTTTTTTTTTTATATTTAAATAAATAGAAGTGTGTAA
GTTCGGTTTGAAAATGGTATTGAGAGTTGTTTTTGTTGGAAATATTTTTTGTTAAGTA
TTTGGGGGTTTTTATTAGTAAGATTTTATTTTAGATGTTTTTTGGTTTTTTTTTTTTAG
GAGTATAGTTATGATTTTAGGTATTTTTTTCGAAAAGAATTTGTTTAATTAAAGGTAA
GTGTATTTTATTTTTATTATGGTTTTTTTTTATTGGGGAAGTAGATAGCGTAGAAAA
AAGAATATATTTATTTTTTATATATTTTTATATTCGTTATATATTTGTTACGTGAGATGT
GTAAAGTTGAATTTAGGGAATGTTTAGTAGTTATATAATAGTGTTATTAAAGGTAATT

GTTTTAGTGATTTTATCGAGTTGGGTTTTGTAAAGATTATAGTATTTTTCGG
TTGTATGTTGGGTATTTTGGAAGTTGTAGTTAATTTGGAGGTTATTAGGGT
ATTATTAGTATATAGTAGTAATTATTGATTAAATGGTGTTTTGGTTTATGTTT
TTTAAGGGGGTTTATTTAGAGGTAGGGTGGAGTTGTTTAGGGTTTTTTTTT
TTTTTTTTTTGTAGACGGAGTTTTGTTTTTGTTGTTTAAGTTGGAGTGTAATG
G (SEQ ID NO:11)

### Exemplary Primer Sequences for Amplification of PAX8/PAX8-AS1 Region of Interest 1 (Chr2: 113994035-113995456):

Forward primer1: TACGGGAGTCGTTTAGGG (SEQ ID NO: 12)
Reverse primer1: CTCAACAACACCCTAAAC (SEQ ID NO: 13)
Forward primer2: **ATTTTGGTTGGATGGTTGGT** (SEQ ID NO: 14)
Reverse primer2: **ACAACCGAAAAATCCCGAAT** (SEQ ID NO: 15)
Forward primer3: **TGGGGAAGTAGATAGCGTAGAAA** (SEQ ID NO: 16)
Reverse primer3: **CCCTAAACAACTCCACCCTACC** (SEQ ID NO: 17)

### LHX6

### LHX6 Region of Interest 1: chr9:124,989,052-124,989,551 5'pad=250 3'pad=250 Non-Modified LHX6 Region of Interest 1 Sequence:

CGGCTCAGCGCGGGCCTCTTTCACTCCGGGCCCCAGCCTTCCCGGTCTCATTT
ACAGTCAGAGGCGCTGAAGCTCAGAAGGTGCATGCGATTCCCCTATTTTTCT
CCCGTAATACTGAGACTCAGGGAAATGGAAATAAACTCTTCTTATTTTTCAG
ACGGAACCCGGGGGCTCAGGCAGACCCTAAGTCTTGCCCAAAGCTTCGCAGT
CGGGCAGCAGTGGAGCCAGGATTTGGAAGTAAGAGGACCTGCGGTGCTTCC
CTGCAGTCTCCTGCGCTGCGTCCCACGCCCCGACAACACGCACGCAACACCT
ACCCCTGTCTCACCTTGAGCAGATATCGGTCCAGGATCTCGAGGCCGCAGCT
GGAGCAGATGTTCTTGCCTGCAGACGGCACGGAGGAGGCGGCAGAGGGCGG
TGAGCAGACAGATGGCGTGCTGGGCGTACATGGGGAGGCCTGACCCTCGTCC
TTGTCCAGAGCTCCTGCCAGGGCCTCGGCGTCAGACTGAGCCTGCGGCGGGG
GAGAGAAGGAGAGGAGCGCTGATCCGGGCACCCAGAGTTGGGGCTGCCTTG
GAGACAAATCTGGGGCCCCCGAGCACCAATTGACAACGAAATCTCCCCAGG
ACAATGCGCCGTAGACTGAAGGACAGGGATGGAAGGGCCTATTGGAGAGGA
AGGGACGGCCCCAACTTTTAACACGCGCATTCTGGGTGCTGGGAGCGGTTAA
AGCCAACATTCCCTGCCGGAAAACCGAGGCTCAGAGAGGGGCGGCGCCCGC
CGGGAGTCCCCCAGAGCGTGAGCAGCAGTTCGTGGCCGGAAAACCTGGCCA

GGTCCCCAGGGCCGGGCCGCTGGAGGACTGGGCACCGCAGGCAGGGCCAAA
CGGACTCACCATGGCGGGCGGCGCGGTCCCTTCAAGACAGCGGGTGGTCGCT
TTGCAGCCGGACCCTGGCTGGGCCATCACCTGGGGGAGGGGGGGAGGGAAC
GCAGGCGGCGGCGGCTGCTG (SEQ ID NO:18)

### Bisulfite-Modified LHX6 Region of Interest 1 Sequence:

CGGTTTAGCGCGGGTTTTTTTTATTTCGGGTTTTAGTTTTTTCGGTTTTATTTA
TAGTTAGAGGCGTTGAAGTTTAGAAGGTGTATGCGATTTTTTTATTTTTTTTT
CGTAATATTGAGATTTAGGGAAATGGAAATAAATTTTTTTTATTTTTTAGACG
GAATTCGGGGGTTTAGGTAGATTTTAAGTTTTGTTTAAAGTTTCGTAGTCGGG
TAGTAGTGGAGTTAGGATTTGGAAGTAAGAGGATTTGCGGTGTTTTTTTGTAG
TTTTTTGCGTTGCGTTTTACGTTTCGATAATACGTACGTAATATTTATTTTTGTTTTAT
TTTGAGTAGATATCGGTTTAGGATTTCGAGGTCGTAGTTGGAGTAGATGTTTTTGTT
TGTAGACGGTACGGAGGAGGCGGTAGAGGGCGGTGAGTAGATAGATGGCGTGTT
GGGCGTATATGGGGAGGTTTGATTTTCGTTTTTGTTTAGAGTTTTTGTTAGGGTTT
CGGCGTTAGATTGAGTTTGCGGCGGGGGAGAGAAGGAGAGGAGCGTTGATTCGG
GTATTTAGAGTTGGGGTTGTTTTGGAGATAAATTTGGGGTTTTCGAGTATTAATTGA
TAACGAAATTTTTTTAGGATAATGCGTCGTAGATTGAAGGATAGGGATGGAAGGGT
TTATTGGAGAGGAAGGGACGGTTTTAATTTTTAATACGCGTATTTTGGGTGTTGGG
AGCGGTTAAAGTTAATATTTTTTGTCGGAAAATCGAGGTTTAGAGAGGGGCGGCG
TTCGTCGGGAGTTTTTTAGAGCGTGAGTAGTAGTTCGTGGTCGGAAAATTTG
GTTAGGTTTTTAGGGTCGGGTCGTTGGAGGATTGGGTATCGTAGGTAGGGTTA
AACGGATTTATTATGGCGGGCGGCGCGGTTTTTTTAAGATAGCGGGTGGTCG
TTTTGTAGTCGGATTTTGGTTGGGTTATTATTTGGGGGAGGGGGGGAGGGAA
CGTAGGCGGCGGCGGTTGTTG (SEQ ID NO:19)

### Exemplary Primer Sequences for Amplification of LHX6 Region of Interest 1:

Forward primer A: GGAATTCGGGGGTTTAGGTA (SEQ ID NO:20)
Reverse primer A: ACGCTCCTCTCCTTCTCTCC (SEQ ID NO:21)
Forward primer B: **GCGTATATGGGGAGGTTTGA** (SEQ ID NO:22)
Reverse primer B: **CCTACGATACCCAATCCTCCAAC** (SEQ ID NO:23)

### LHX6 Region of Interest 2: chr9:124985156-124990089 5'pad=250 3'pad=250 Non-Modified LHX6 Region of Interest 2 Sequence:

TTGGGAGCTCTTGGCCTCTCTTTGGGCAGAGTGCTCCAGTCCACATCCTACCA
GACCCTTGACCCCGGGCTTCAGGAAACAGAAGCCTAGAGAACCTCAGCATGT
GACCCCTCCCTGGGCCCCAGCTCCTTAGAGGAAAATTCTGCCTCCCACGCCA
CCATTCACACTCTCTCCAGGAAGAACTTCCTCATAGCAGCCACCCAGAGCTT
GGGCTGCAGTGCAGTGCAGACAATAGTCCTTGCCTGGCACTCGATCGGCTTT
TTCTTTCCCTTTTTGTGGGTTTTATTATTTTTGTTACAGGTGGGGCTGCCAGGG
ATGGGTTATGCCTGCAGGGGGAGAAATGATCAGAAGAGCCACAATTCTACC
CTCAACCCAGGGAATGTGAGCTTTTACTGCCTCCATTTTCTTAACCAGAAATC
TGGTTTAGTTTTGCTTTCAGGGCTCTTGTCATTGGAAAAAGATTAGTGCTCAG
AACCTGCAGCCCAGAAATGGCCCAGTCCCATCCCTCCAGGCAGGCTCTGCTC
CTCAAGCTGCGTGGGGAAATGCCATCATGGGCTTGTGCCCAGCATCCACCCT
GCCAATGATGACAACAGTGATAATCGAGGCATTCATACCAACCACCATTTAG
GGAGCCTTTATAGAGACTTCCCCCCAACAGTGGCCTCTTTGGCAGCTCTGGG
AGACAGCCTTGGAAAGGGCGAACTAAGGGCCGAGAGAAGCAGCAACTTGCG
AAGGTCACTACTGGCCAGACTGGGCCCAGTCTCCTGCCTCTTCCCACAGTGG
CTGCACACCCTACTCTCTACCCCATTGCAGTAGGAAGAGAACTCCAGAATAA
GGGGAGGCTCTGAACAGTGGAGTCAGGATCCGTGTTTTGACTAGTTTCAAGG
TCTAGTAAAGCACAGTATCCAAGGAGGCTCAGTCCAGCATGGGCATGGGCTA
GGGCTGGAGAAGTAGAAAAGAAATTTGGGCAAGTAGTGAATAAGACTGCCA
AATCTATTGTTAGGAGAGCCAGGAGAGGTGTCCTAGTGGTGCCTTAGTCTAT
GGGAGAGACCCAGACACCATTCTGCTGGGTTCCCAAGCTGGCACCTTAAATT
TAGTAGCTGATGAATCCAAGGAGCCCCCAAGCATCATCTGGCATGTTTTTTT
GTATGTTTGTTTGTTTGTTTTTTGAGACAGAGTCTCTCTCTGTTACCCAGGCTG
GAGTGTAGTGACGTGATCTTGGCTCACCACAATGTCCGCCTCCCAGGTTCAA
GTGATTCTCTTGCCTCAGCCTCCTGAGTAGCTGGGACTACAGGCACGTGCCA
CCACACCTGGCTAAGTTTTGTATTTTTAGTAGAGATGGGTTTCACCATGTTGG
CCAGGATGGTCTCGATCTCCTGACCTTGTGATCTACCAGCCTTGGCCTCCCAA
AGTGCTGCGATTATAGGTGTGAGCCACCACACCCGGCCCATCTGGCATGTTT
TATACCAACTGGAGTCAGGCCTAGAGCAGGGAGGTGGCTAGACAGTGACTG
TAACCTCCATTTCCTGTCCTGGCCAAACCAATACTCAGATGGGACAGTGAGA
GGAGACATCACACTCTGGTTACACACCATTAACCCCTGTCTGACTACACACA
CACACACACACAGCCGTCCAGATTCACAAATACGTTCTTATCACCCCCTTCAT
CCCTCAGTGCCTGGGTGGAGGCCCTTAAGCAGGAATGCCCCCTCAGAGGAGA
GGGTGGGAGTGGAATGATGGGATTTCAGAATCCCCTCACCTCACCCTCTCAC
CTCCAGATCCTAGTCCAGATGTAATCCTGTCCTCACCACTTTGCGTGCACGTG

CACGCACACACACATGCACACACACAGTCTCAACATTCACATACAGACACCC
ACTCTTGACACCCACACATGAACTAAAAGACAAACGTTGGTCCATAAAGGA
ACACACATGCTATCTACATACCCCCAACCCTGCCCAGTGATGCTTTCCCCAGC
ACTACTGCACAGATCCCAGACAATACAGACACACAGGCACTCCTTGCACACT
GAGTCCTAGGCCACTTGGTTGAGCTGCCCCCACCCCACCCCAGCTGCACACT
CTTCTACATCCATCCCATAAAGGAAGAGTCCGTCTCTTTAACCATGCTTCTCA
TGGTTTTCAGAGTACCCTTGCCCCAAATGCTGCCAACCTCCCCCTAGACTCAA
GATCTTCTCTTTCCAGATCGCTGGAGGCTCAATGCCAGTGCACAGGAGGAGA
TCGCCTTCCACCATATTCCCTGACTCCCAGAGGCTCTTTCTAGGGCAGGGGTC
TGGCCAGGAAGACTGAAGCCATGACGTCTTCCTGTGCAAGGATAGGAAAGA
TGTCTGTGGGTTGGAGGGTAGTTATAGGGAAGCAGCTTTCTCCACTTTTTGAG
AAAGCACTTGGGAACAAGCATCCCTCTCAGGAAGTGATTCTCCACCATTCTC
CTCTGATCCTATAGGCCTGGTGGTCCAAGCCTCAGGCCAGTTGATGCCTCCC
ACTGGGATCAGCAGACAAGGCCATGACCCTGCAGTGACCTCGACCCCATAAT
CAGGTCAGGTCCGGGCTTCATGGCTAGAAGCGAGGCCCAGGCGAGTGGCCA
AGACGCGGCAGGACAGGAGGATGCGCCTGGCTGCTCTCGGCTGCTGGACGC
GGCCTGCAGTAGGGAGAGCGCAGCCCGGGGTAATCCCACTCAGGGCTCCGG
GTGCCCAGTTCTCCCACTTCCCATCCTGGCGGCCTAAAGGCAAGGCCTAGAC
CCTGGAGAGGCCAAAGTCGGGCCAAAACAACAGCGAAGCTGGGACTGGAAC
AGGCGCTGGGGGCCTGCGCATGACCCCAGGCTCAGAATGCGACACTCCAGT
GACGCCGGGGAATGAGTTGAGATGGAGCCGGAGACAGAGGTTGGGGTTCAG
GACGAGGCGCTTGGGTATGGGATCGGATCTGAGACGGTTGCGGCGCTGGTGC
TTTGGGCTGGACCAGACTCGAACCCAGAGCGGAGCGAGGGTCAGAGCCAGG
GCAAGAGTCCGAAGCCCAGCTCAGCGTCCAGCCCGCGAGAGACCCCACCAT
GAATGACGTCCGAGTAGGGTTGGAACTCGAAGCCCGAACGCACTGGAAATG
CAGCCGGACGACGGCTGGGATCCGAGTGGGTCCGGGCCAGAAATGGGGACC
TCAGAGCTCCACCGAGGCGCTCGAGGTCAGAACTGAAGCCTGAGACTTAGG
CAGGACCCGAGACAGAGCCAGAGACGATACCGAAACCCAATGGACCGCGAG
GACCGAAGGCAGATCCGGGGCGCAAACCTGTGCAGGCACTGGCGCGCTGCC
TGGAGCTCTGGGTTCGCGCCGCTGAGCGCCGGCAGGTTGGACCAGCGCTGCG
CGCCGGAAGTGCACTCGGGACGCCGGGGTTCTGGAGGAGAGACCACACGCC
GCAAAAGTGGCCTCCGAATGCGCCCGGGGCCTGCCCTCGGCGACACTGCTGG
CTCGGCGGCCGCAGCCTACCTGAAGTAGTCCATCTTGCAGAAGATCTCCTTG
TTCTTGATGTAGCAGCTGTTCTGCTGCCTCAGCGACGTGCGACACACGGAGC
ACTCGAGGCACCGCACGTGCCAGATGAGGTTGTTGACCTGGGGACGGGGCG

GGGACGGAGGTCGGCTCAGCGCGGGCCTCTTTCACTCCGGGCCCCAGCCTTC
CCGGTCTCATTTACAGTCAGAGGCGCTGAAGCTCAGAAGGTGCATGCGATTC
CCCTATTTTTCTCCCGTAATACTGAGACTCAGGGAAATGGAAATAAACTCTTC
TTATTTTTCAGACGGAACCCGGGGGCTCAGGCAGACCCTAAGTCTTGCCCAA
AGCTTCGCAGTCGGGCAGCAGTGGAGCCAGGATTTGGAAGTAAGAGGACCT
GCGGTGCTTCCCTGCAGTCTCCTGCGCTGCGTCCCACGCCCCGACAACACGC
ACGCAACACCTACCCCTGTCTCACCTTGAGCAGATATCGGTCCAGGATCTCG
AGGCCGCAGCTGGAGCAGATGTTCTTGCCTGCAGACGGCACGGAGGAGGCG
GCAGAGGGCGGTGAGCAGACAGATGGCGTGCTGGGCGTACATGGGGAGGCC
TGACCCTCGTCCTTGTCCAGAGCTCCTGCCAGGGCCTCGGCGTCAGACTGAG
CCTGCGGCGGGGGAGAGAAGGAGAGGAGCGCTGATCCGGGCACCCAGAGTT
GGGGCTGCCTTGGAGACAAATCTGGGGCCCCCGAGCACCAATTGACAACGA
AATCTCCCCAGGACAATGCGCCGTAGACTGAAGGACAGGGATGGAAGGGCC
TATTGGAGAGGAAGGGACGGCCCCAACTTTTAACACGCGCATTCTGGGTGCT
GGGAGCGGTTAAAGCCAACATTCCCTGCCGGAAAACCGAGGCTCAGAGAGG
GGCGGCGCCCGCCGGGAGTCCCCCAGAGCGTGAGCAGCAGTTCGTGGCCGG
AAAACCTGGCCAGGTCCCCAGGGCCGGGCCGCTGGAGGACTGGGCACCGCA
GGCAGGGCCAAACGGACTCACCATGGCGGGCGGCGCGGTCCCTTCAAGACA
GCGGGTGGTCGCTTTGCAGCCGGACCCTGGCTGGGCCATCACCTGGGGGAGG
GGGGGAGGGAACGCAGGCGGCGGCTGCTGAACTGGCTCCGCACAGCCT
GAGCCCAGCGCCTCCCCGCGCCTGTTATATAAACCGGCGCCGAACAATGAGT
CCTAACTTTGTAGTGGGCATTTAAAGCCCTTCCCCACAAAAGCGGTGTCTCTC
TAATGAAGCAATTTGAATTTGGATTGGATTTTTTCCCTCTCTCTCCCCCTCTCC
CTGCACTTAACCCGTGGCTCTTGAAGTAATCGCTTAGTTCCCTTGCAATCCAA
GCCTCTGAGGGGGGAAGAAAAACACGCGCACACACACACAAACTACCTGCA
ATTAGA (SEQ ID NO:24)

### Bisulfite-Modified LHX6 Region of Interest 2 Sequence:

TTGGGAGTTTTTGGTTTTTTTTTGGGTAGAGTGTTTTAGTTTATATTTTATTAG
ATTTTTGATTTCGGGTTTTAGGAAATAGAAGTTTAGAGAATTTTAGTATGTGA
TTTTTTTTTGGGTTTTAGTTTTTTAGAGGAAAATTTTGTTTTTTACGTTATTATT
TATATTTTTTTTAGGAAGAATTTTTTTATAGTAGTTATTTAGAGTTTGGGTTGT
AGTGTAGTGTAGATAATAGTTTTTGTTTGGTATTCGATCGGTTTTTTTTTTTTTTT
TTTGTGGGTTTTATTATTTTTGTTATAGGTGGGGTTGTTAGGGATGGGTTATGTTTG
TAGGGGGAGAAATGATTAGAAGAGTTATAATTTTATTTTTAATTTAGGGAATGTGAG

TTTTTATTGTTTTATTTTTTAATTAGAAATTTGGTTTAGTTTTGTTTTTAGGGTTTTT
GTTATTGGAAAAAGATTAGTGTTTAGAATTTGTAGTTTAGAAATGGTTTAGTTTTATT
TTTTTAGGTAGGTTTTGTTTTTTAAGTTGCGTGGGGAAATGTTATTATGGGTTTGTG
TTTAGTATTTATTTTGTTAATGATGATAATAGTGATAATCGAGGTATTTATATTAATTA
TTATTTAGGGAGTTTTTATAGAGATTTTTTTTTAATAGTGGTTTTTTTGGTAGTTTTGG
GAGATAGTTTTGGAAAGGGCGAATTAAGGGTCGAGAGAAGTAGTAATTTGCGAAG
GTTATTATTGGTTAGATTGGGTTTAGTTTTTTGTTTTTTTTTATAGTGGTTGTATATTT
TATTTTTTATTTTATTGTAGTAGGAAGAGAATTTTAGAATAAGGGGAGGTTTTGAATA
GTGGAGTTAGGATTCGTGTTTTGATTAGTTTTAAGGTTTAGTAAAGTATAGTATTTAA
GGAGGTTTAGTTTAGTATGGGTATGGGTTAGGGTTGGAGAAGTAGAAAAGAAATTT
GGGTAAGTAGTGAATAAGATTGTTAAATTTATTGTTAGGAGAGTTAGGAGAGGTGT
TTTAGTGGTGTTTTAGTTTATGGGAGAGATTTAGATATTATTTTGTTGGGTTTTTAAG
TTGGTATTTTAAATTTAGTAGTTGATGAATTTAAGGAGTTTTTAAGTATTATTTGGTAT
GTTTTTTTGTATGTTTGTTTGTTTGTTTTTTGAGATAGAGTTTTTTTTTGTTATTTAGG
TTGGAGTGTAGTGACGTGATTTTGGTTTATTATAATGTTCGTTTTTTAGGTTTAAGTG
ATTTTTTTGTTTTAGTTTTTTGAGTAGTTGGGATTATAGGTACGTGTTATTATATTTG
GTTAAGTTTTGTATTTTTAGTAGAGATGGGTTTTATTATGTTGGTTAGGATGGTTTCG
ATTTTTTGATTTTGTGATTTATTAGTTTTGGTTTTTTAAAGTGTTGCGATTATAGGTGT
GAGTTATTATATTCGGTTTATTTGGTATGTTTTATATTAATTGGAGTTAGGTTTAGAG
TAGGGAGGTGGTTAGATAGTGATTGTAATTTTTATTTTTTGTTTTGGTTAAATTAATA
TTTAGATGGGATAGTGAGAGGAGATATTATATTTTGGTTATATATTATTAATTTTTGT
TTGATTATATATATATATATATATAGTCGTTTAGATTTATAAATACGTTTTTATTATTTT
TTTTATTTTTTAGTGTTTGGGTGGAGGTTTTTAAGTAGGAATGTTTTTTTAGAGGAGA
GGGTGGGAGTGGAATGATGGGATTTTAGAATTTTTTTATTTTATTTTTTTATTTTTAG
ATTTTAGTTTAGATGTAATTTTGTTTTTATTATTTTGCGTGTACGTGTACGTATATATA
TATGTATATATATAGTTTTAATATTTATATATAGATATTTATTTTTGATATTTATATATG
AATTAAAAGATAAACGTTGGTTTATAAAGGAATATATATGTTATTTATATATTTTTAAT
TTTGTTTAGTGATGTTTTTTTTAGTATTATTGTATAGATTTTAGATAATATAGATATAT
AGGTATTTTTTGTATATTGAGTTTTAGGTTATTTGGTTGAGTTGTTTTTATTTTATTTT
AGTTGTATATTTTTTTATATTTATTTTATAAAGGAAGAGTTCGTTTTTTTAATTATGTTT
TTTATGGTTTTTAGAGTATTTTTGTTTTAAATGTTGTTAATTTTTTTTTAGATTTAAGAT
TTTTTTTTTTTAGATCGTTGGAGGTTTAATGTTAGTGTATAGGAGGAGATCGTTTTTT
ATTATATTTTTTGATTTTTAGAGGTTTTTTTTAGGGTAGGGGTTTGGTTAGGAAGATT
GAAGTTATGACGTTTTTTTGTGTAAGGATAGGAAAGATGTTTGTGGGTTGGAGGGT
AGTTATAGGGAAGTAGTTTTTTTTATTTTTTGAGAAAGTATTTGGGAATAAGTATTTT

TTTTAGGAAGTGATTTTTTATTATTTTTTTTTGATTTTATAGGTTTGGTGGTTTAAGTT
TTAGGTTAGTTGATGTTTTTTATTGGGATTAGTAGATAAGGTTATGATTTTGTAGTGA
TTTCGATTTTATAATTAGGTTAGGTTCGGGTTTTATGGTTAGAAGCGAGGTTTAGGC
GAGTGGTTAAGACGCGGTAGGATAGGAGGATGCGTTTGGTTGTTTTCGGTTGTTG
GACGCGGTTTGTAGTAGGGAGAGCGTAGTTCGGGGTAATTTTATTTAGGGTTTCG
GGTGTTTAGTTTTTTTATTTTTTATTTTGGCGGTTTAAAGGTAAGGTTTAGATTTTGG
AGAGGTTAAAGTCGGGTTAAAATAATAGCGAAGTTGGGATTGGAATAGGCGTTGG
GGGTTTGCGTATGATTTTAGGTTTAGAATGCGATATTTTAGTGACGTCGGGGAATG
AGTTGAGATGGAGTCGGAGATAGAGGTTGGGGTTTAGGACGAGGCGTTTGGGTAT
GGGATCGGATTTGAGACGGTTGCGGCGTTGGTGTTTTGGGTTGGATTAGATTCGA
ATTTAGAGCGGAGCGAGGGTTAGAGTTAGGGTAAGAGTTCGAAGTTTAGTTTAGC
GTTTAGTTCGCGAGAGATTTTATTATGAATGACGTTCGAGTAGGGTTGGAATTCGA
AGTTCGAACGTATTGGAAATGTAGTCGGACGACGGTTGGGATTCGAGTGGGTTCG
GGTTAGAAATGGGGATTTTAGAGTTTTATCGAGGCGTTCGAGGTTAGAATTGAAGT
TTGAGATTTAGGTAGGATTCGAGATAGAGTTAGAGACGATATCGAAATTTAATGGA
TCGCGAGGATCGAAGGTAGATTCGGGGCGTAAATTTGTGTAGGTATTGGCGCGTT
GTTTGGAGTTTTGGGTTCGCGTCGTTGAGCGTCGGTAGGTTGGATTAGCGTTGCG
CGTCGGAAGTGTATTCGGGACGTCGGGGTTTTGGAGGAGAGATTATACGTCGTAA
AAGTGGTTTTCGAATGCGTTCGGGGTTTGTTTTCGGCGATATTGTTGGTTCGGCGG
TCGTAGTTTATTTGAAGTAGTTTATTTTGTAGAAGATTTTTTTGTTTTTGATGTAGTAG
TTGTTTTGTTGTTTTAGCGACGTGCGATATACGGAGTATTCGAGGTATCGTACGTG
TTAGATGAGGTTGTTGATTTGGGGACGGGGCGGGGACGGAGGTCGGTTTAGCG
CGGGTTTTTTTTATTTCGGGTTTTAGTTTTTTCGGTTTTATTTATAGTTAGAGGCGTT
GAAGTTTAGAAGGTGTATGCGATTTTTTTATTTTTTTTTCGTAATATTGAGATTTAGG
GAAATGGAAATAAATTTTTTTTATTTTTTAGACGGAATTCGGGGGTTTAGGTAGATTT
TAAGTTTTGTTTAAAGTTTCGTAGTCGGGTAGTAGTGGAGTTAGGATTTGGAAGTAA
GAGGATTTGCGGTGTTTTTTTGTAGTTTTTTGCGTTGCGTTTTACGTTTCGATAATA
CGTACGTAATATTTATTTTTGTTTTATTTTGAGTAGATATCGGTTTAGGATTTCGAGG
TCGTAGTTGGAGTAGATGTTTTTGTTTGTAGACGGTACGGAGGAGGCGGTAGAGG
GCGGTGAGTAGATAGATGGCGTGTTGGGCGTATATGGGGAGGTTTGATTTTCGTT
TTTGTTTAGAGTTTTTGTTAGGGTTTCGGCGTTAGATTGAGTTTGCGGCGGGGGAG
AGAAGGAGAGGAGCGTTGATTCGGGTATTTAGAGTTGGGGTTGTTTTGGAGATAA
ATTTGGGGTTTTCGAGTATTAATTGATAACGAAATTTTTTTAGGATAATGCGTCGTA
GATTGAAGGATAGGGATGGAAGGGTTTATTGGAGAGGAAGGGACGGTTTTAATTTT
TAATACGCGTATTTTGGGTGTTGGGAGCGGTTAAAGTTAATATTTTTTGTCGGAAA

ATCGAGGTTTAGAGAGGGGCGGCGTTCGTCGGGAGTTTTTTAGAGCGTGAGTAGT
AGTTCGTGGTCGGAAAATTTGGTTAGGTTTTTAGGGTCGGGTCGTTGGAGGATTG
GGTATCGTAGGTAGGGTTAAACGGATTTATTATGGCGGGCGGCGGTTTTTTAA
GATAGCGGGTGGTCGTTTTGTAGTCGGATTTTGGTTGGGTTATTATTTGGGGGAG
GGGGGGAGGGAACGTAGGCGGCGGCGGTTGTTGAATTGGTTTCGTATAGTTTGA
GTTTAGCGTTTTTTCGCGTTTGTTATATAAATCGGCGTCGAATAATGAGTTTT
AATTTTGTAGTGGGTATTTAAAGTTTTTTTTTATAAAAGCGGTGTTTTTTTAAT
GAAGTAATTTGAATTTGGATTGGATTTTTTTTTTTTTTTTTTTTTTTTTTTTGTA
TTTAATTCGTGGTTTTTGAAGTAATCGTTTAGTTTTTTTGTAATTTAAGTTTTT
GAGGGGGGAAGAAAAATACGCGTATATATATATAAATTATTTGTAATTAGA
(SEQ ID NO:25)

### Exemplary Primer Sequences for Amplification of LHX6 Region of Interest 2 (Chr9: 124985406-124989839):

Forward primer1: TTGGGTTGTAGTGTAGTGTAGA (SEQ ID NO:26)
Reverse primer1: AATTCGCCCTTTCCAAAACT (SEQ ID NO:27)
Forward primer2: **GAGAGCGTAGTTCGGGGTAA** (SEQ ID NO:28)
Reverse primer2: **TCTCGCGAACTAAACGCTAAA** (SEQ ID NO:29)
Forward primer3: **AATTTAATGGATCGCGAGGA** (SEQ ID NO:30)
Reverse primer3: **CCCCAAATCAACAACCTCAT** (SEQ ID NO:31)
Forward primer4: **GGTGTTGGGAGCGGTTAAAG** (SEQ ID NO:32)
Reverse primer4: **AAAACTCATTATTCGACGCC** (SEQ ID NO:33)

### GABRA2

### GABRA2 Region of Interest: chr4:46,391,392-46,391,695 5'pad=250 3'pad=250 Non-Modified GABRA2 Region of Interest Sequence:

GAGAGATGGGTACTTCACGCCACAACCCCCTCAACCAAGACCACCTCCGCAA
CCACAGCGGGAAAAGCTAGGGGCAGGCAGCCCACTTTTGCAATTACTCTACA
TTACAGTGAACTGCGGTCCTCACGTCTTCTTTTCTTCACTCCCCTTAACAAAA
TAAAATAAATAATTTCCCTTCCTGCCCACCCACGGTTGCCCAAGACCAAGTC
TTAGAGGCAGCCGGGTTCCGATCAAGTGCTGCGAAACGACGCGTTTGACAGC
TGCTCTGGAGCCGAGGATCACAATAAGAGAAGGCGCGTGAGGCTCCGGCAG
CAAACACCAGCCAGGCAGGCAGCGGTAATCACAGTGAGCAAAGTTGAAGAC
TATAAAAGAGCCAGGCTAACGTGCTGCCGAGCAGGTGTCCTGGATTTTATTG
TAGTTGCAAATATGCTTCTGGTGATAAGTATCGAAATTATTTTTTAAGTGCGC

TGGGGAGGAGGGAGGTGCGGGAATTGAGCCTCCTCCGTTTCCAGGGAGCCTC
TGAAAGTGGGGTGGGGGGAGATGAGGCATGCACGCGAGGCAAAGACAAAC
AGGAAAGTGTCTCTATCGGGACCAACGTGTGCGACCCTACCTGAAACGGCAA
GCAGAATTCGGTGTTTTCTTCCTTTTGCCCTGATCTTGACGAGATAGGAAACT
TGGGAGAGAGAGAGAGAGAGAGAGAGAGAGAGAGAGAGAGAGAGAGAGA
GAGAGAGAGAGAGACCGAGACTGCAGCAGCCAAGAGAGCGTGGAGCGATG
GGCTGGTGGAAGCCGGAGAGGAGCGCTAGG (SEQ ID NO:34)

### Bisulfite-Modified GABRA2 Region of Interest Sequence:

GAGAGATGGGTATTTTACGTTATAATTTTTTTAATTAAGATTATTTTCGTAAT
TATAGCGGGAAAAGTTAGGGGTAGGTAGTTTATTTTTGTAATTATTTTATATT
ATAGTGAATTGCGGTTTTTACGTTTTTTTTTTTTTATTTTTTTTAATAAAATAA
AATAAATAATTTTTTTTTTTGTTTATTTACGGTTGTTTAAGATTAAGTTTTAGA
GGTAGTCGGGTTTCGATTAAGTGTTGCGAAACGACGCGTTTGATAGTTGTTTTG
GAGTCGAGGATTATAATAAGAGAAGGCGCGTGAGGTTTCGGTAGTAAATATTAGTT
AGGTAGGTAGCGGTAATTATAGTGAGTAAAGTTGAAGATTATAAAAGAGTTAGGTT
AACGTGTTGTCGAGTAGGTGTTTTGGATTTTATTGTAGTTGTAAATATGTTTTTGGT
GATAAGTATCGAAATTATTTTTTAAGTGCGTTGGGGAGGAGGGAGGTGCGGGAATT
GAGTTTTTTTCGTTTTTAGGGAGTTTTTGAAAGTGGGGTGGGGGGAGATGAGGTAT
GTACGCGAGGTAAAGATAAATAGGAAAGTGTTTTTATCGGGATTAACGTGTG
CGATTTTATTTGAAACGGTAAGTAGAATTCGGTGTTTTTTTTTTTTTGTTTTGA
TTTTGACGAGATAGGAAATTTGGGAGAGAGAGAGAGAGAGAGAGAGAGAG
AGAGAGAGAGAGAGAGAGAGAGAGAGAGAGAGATCGAGATTGTAGTAGTT
AAGAGAGCGTGGAGCGATGGGTTGGTGGAAGTCGGAGAGGAGCGTTAGG
(SEQ ID NO:35)

### Exemplary Primer Sequences for Amplification of GABRA2 Region of Interest:

Forward primer: TCGGGTTTCGATTAAGTGTTG (SEQ ID NO:36)
Reverse primer: TTTCAAATAAAATCGCACAC (SEQ ID NO:37)

In some embodiments, the biomarker panel is a microarray.

In some embodiments, the primers or probes are biotinylated and bind to streptavidin coated substrates for selection of the DMPs or DMP-associated genes targeted by the probe or primers. In some embodiments, the streptavidin-coated substrates are beads.

Described herein are methods to assay the methylation status of DMPs or DMP-associated genes described herein to diagnose or give a prognosis for hypersomnolence, idiopathic hypersomnia, or long sleep time in an individual. Methylation levels of at least one DMP or DMP-associated gene recited in Tables 1 and 2, or any combination of DMPs or DMP-associated genes recited therein, is measured in target DNA from a biofluid sample from a subj ect.

Methylation may be quantified by any suitable means known in the art. Suitable methods for assaying quantification are disclosed, for example, by Kurdyukov and Bullock ("DNA methylation analysis: Choosing the right method," Biology, 2016, 5(3)). Suitable methods for quantifying or assaying methylation may include, but are not limited to methylation specific polymerase chain reaction (PCR), high resolution melting, cold-PCR, pyrosequencing, PCR and sequencing, bead array, and digestion-based assay followed by PCR or quantitative PCR (qPCR).

In some embodiments, the target DNA is bisulfite modified. Bisulfite treatment mediates the deamination of cytosine to uracil, whereby the modified uracil residue will be read as a thymine as determined by PCR-amplification and sequencing. Methylated cytosine (5mC) resides are protected from this conversion and will remain as cytosine.

To examine the methylation status of the DMP or DMP-associated gene, target genomic DNA may be isolated from a biofluid sample from a subject. In some embodiments, the target DNA is isolated from a biofluid sample from a human.

As used herein, "biofluid" refers to a biological fluid produced by a subject. Suitable biofluids include, but are not limited to blood, saliva, sweat, urine, semen, tears, and cerebrospinal fluid. In some embodiments, the biofluid is selected from the group consisting of blood, saliva, sweat, urine, semen, tears, and cerebrospinal fluid. In some embodiments, the biofluid is blood. In some embodiments, the biofluid is saliva.

Following isolation of target DNA, the target DNA will be contacted with probes specific to the DMPs outlined in Tables 1 and 2 to isolate and enrich these genomic regions from the target DNA sample. In some embodiments, sequences of the DMP is used as bait to isolate the genomic regions of interest for amplification and sequencing.

After isolation and enrichment of the genomic regions within the target DNA that include the DMP, methylated adapters are ligated to the enriched regions. The sample with the ligated methylated adapters may then be subject to sodium bisulfite modification.

In some embodiments, the target DNA sample is bisulfite modified without first enriching the sample of the genomic regions of the DMPs. In some embodiments, the target DNA is first bisulfite modified then enriched for the genomic regions of the DMPs.

In general, target DNA or bisulfite modified target DNA is subject to amplification. The amplification may be polymerase chain reaction (PCR) amplification. PCR amplification will include single or multiple pair(s) of primers and probes at specific DMPs outlined in Tables 1 and 2. The target DNA amplification and methylation quantification will be evaluated in one or multiple tubes.

In some embodiments, methylation is quantified by amplification and sequencing of target DNA. Bisulfite modified target DNA may be subject to PCR to amplify target regions comprising DMPs outlined in Tables 1 and 2. The PCR reaction mixture typically includes at least one pair of primers designed to target a DMP detailed in Tables 1 and 2, PCR buffer, dNTPs (e.g., adenine, thymine, cytosine and guanine), MgCl₂, and polymerase. PCR amplification generally includes the steps of heating the reaction mixture to separate the strands of the target DNA, annealing the primers to the target DNA by cooling the reaction mixture, allowing the polymerase to extend the primers by addition of NTPs, and repeating the process at least 2, at least 5, at least 10, at least 15, at least 20, at least 25, or at least 30 times to produce a PCR amplification product. If the target DNA in the reaction mixture is single stranded, the initial heating step may be omitted, however this heating step will need to be included when the second and subsequent times the reaction is completed to separate the extended primer strands from the opposite strand and DNA (e.g., the target DNA or another previously extended primer strand). In some embodiments, the target DNA is bisulfite modified prior to amplification.

In some embodiments, the bisulfite modified target DNA is used in a methylation-specific-quantitative PCR (MS-QPCR) reaction such as MethylLight (WO 2000/070090A1) or HeavyMethyl (WO 2002/072880A2). For example, a reaction mixture for use in a MethylLight methylation specific PCR reaction would contain primers and probes specific to the DMPs recited in Tables 1 and 2, PCR buffer, dNTPs (e.g., adenine, thymine, cytosine and guanine), MgCl₂, and polymerase. A typical kit for methylation specific PCR may include primers and probes specific to the DMPs recited in Tables 1 and 2, wild type reference gene primers such as β-actin, PCR buffer, dNTPs, MgCl₂, polymerase, positive and negative methylation controls, and a dilution reference. The MS-QPCR may be carried out in one or multiple reaction tubes.

In some embodiments, either the forward or reverse primer of the primer pair used in the PCR amplification reaction is biotinylated. When a biotinylated primer is used in a PCR amplification reaction, PCR products may be purified, captured, and/or sorted with a streptavidin coated substrate. In some embodiments, the substrate is a streptavidin coated bead. In some embodiments, the beads are streptavidin sepharose beads. In some embodiments, the beads are magnetic.

In some embodiments, the PCR amplification product is contacted with one or more probes specific for and complementary to a DMP detailed in Tables 1 and 2. The probe may be biotinylated. The PCR amplification product and probe mixture can then be purified, captured and/or sorted with a streptavidin coated substrate. In some embodiments, the substrate is a streptavidin coated bead. In some embodiments, the beads are streptavidin sepharose beads. In some embodiments, the streptavidin beads are magnetic.

In some embodiments, methylation is quantified using pyrosequencing. Bisulfite modified target DNA may be subject to PCR to amplify target regions comprising DMPs outlined in Tables 1 and 2 as described above. PCR amplification products are purified, denatured to single-stranded DNA, and annealed to a sequencing primer for methylation quantification by pyrosequencing of the DMP or DMP-associated gene as detailed in Tables 1 and 2. In some embodiments, methylation may be quantified with PyroMark^{™}MD Pyrosequencing System (Qiagen) using PyroPyroMark^{®} Gold Q96 Reagents (Qiagen, Cat# 972804) (QIAGEN PyroMark Gold Q96 Reagents Handbook 08/2009, 36-38).

In some embodiments, bisulfite treated DNA is subject to an Invader^{®} assay to detect changes in methylation. The Invader^{®} assay entails the use of Invader^{®} chemistry (Hologic Inc.; invaderchemistry.com; Day, S., and Mast, A. Invader assay, 2004; Chapter in Encyclopedia of Diagnostic Genomics and Proteomics. Marcel Dekker, Inc., U.S. Patent Nos. 7,011,944; 6,913,881; 6,875,572 and 6,872,816). In the Invader^{®} assay, one would use a structure-specific flap endonuclease (FEN) to cleave a three-dimensional complex formed by hybridization of C/T specific overlapping oligonucleotides to target DNA containing a CG site. Initial PCR amplification of the bisulfite treated target DNA may be necessary if the quantity of the bisulfite treated target DNA is less than 20 ng.

In some embodiments, the methylation status is measured by methylation-specific PCR, quantitative methylation-specific PCR, methylation-sensitive DNA restriction enzyme analysis, or bisulfite genomic sequencing PCR, or quantitative bisulfite pyrosequencing. See, *e.g.,* U.S. Patent Nos. 10,370,726 and 10,934,592.

Methylation status of the DMPs and DMP-associated genes described herein may be useful in identification and management of both acute and chronic sleep problems in a subject. Assays described herein are useful for identifying insufficient sleep time in persons complaining of daytime sleepiness improving patient care in sleep medicine. The assays described can also be used as to quantify change in sleep duration in research and clinical care (e.g., identifying increased sleep time caused by a medication; clarifying whether a behavioral intervention increases sleep time in people who are chronically sleep restricted, etc.) or in general assessments of sleep time in a subject.

For applications in general wellness assessments of subject (e.g., a healthy subject), the methylation assays to evaluate sleep in a subject as described herein can be used to optimize mental performance, to optimize physical performance, to optimize recovery after mental exertion, to optimize recovery after physical exertion, to optimize academic performance, to help control weight, to help reduce weight, to help reduce obesity, to help control acute pain, to help control chronic pain, and to prevent the onset of medical, psychiatric, and neurological disease.

Methods described herein for assaying methylation to evaluate sleep in a subject as described herein may be used to increase or optimize fecundity or fertility in a subject. Likewise, the methods described herein for assaying methylation to evaluate sleep in a subject as described herein may be used to aid in a subject's general wellness during pregnancy. The methods described herein for assaying methylation to evaluate sleep in a subject may be used to aid in post-partum recovery and mental and physical wellness post-partum.

In some embodiments, the methods described herein for assaying methylation to evaluate sleep in a subject may be used to increase or optimize wound healing. The methods may also help in physical or mental trauma recovery in an individual or in post-surgical recovery.

In some embodiments, the methods described herein for assaying methylation may be used to evaluate sleep duration and sleepiness in a subject. In some embodiments, the methods described herein for assaying methylation may be used to evaluate sleep duration and sleepiness in a subject diagnosed with a sleep disorder (e.g., insomnia, restless legs syndrome, circadian rhythm sleep-wake disorders, sleep apnea, and parasomnias).

In some embodiments, methods described herein for assaying methylation may be used to evaluate sleep duration in a subject with a medical or neurological disorder (e.g., cancer, coronary heart disease, atrial fibrillation, congestive heart failure, epilepsy/seizure disorders, thyroid dysfunction, Parkinson's disease, fibromyalgia, Myotonic dystrophy, Kline Levin Syndrome, rheumatologic disease such as systemic lupus erythematosus, renal disease, hepatic disease such as non-alcoholic fatty liver disease, diabetes, hypertension, and asthma). In some embodiments, methods described herein for assaying methylation may be used to evaluate sleep duration in a subject with a psychiatric disorder (e.g., mood, thought, attentional, psychotic, personality, anxiety, and PTSD)

Assaying methylation status of the DMPs and DMP-associated genes described herewith will be beneficial as a measure of habitual sleep duration to help subjects identify if they are sleeping too little or too much to maintain personal wellness. In some embodiments, assays for determining the methylation status of DMPs or DMP-associated genes described herein may be incorporated into a person fitness tracker and the target DNA may be from sweat.

Assaying methylation status of the DMPs and DMP-associated genes described herein may also be beneficial for use in employee health screening (e.g., to determine if an employee is at risk for chronic disease), for use in actuarial sciences and risk estimation (e.g., to determine if an individual has sleep insufficiencies or excessive sleep times that would be associated with increased mortality), to monitor sleep and its impact on chronic disease treatment (e.g., identifying and characterizing sleep duration can improve management of chronic illnesses such as diabetes, asthma, and hypertension), identification of individuals who may be at risk for psychological disorders or mental illness (e.g. too much or too little sleep are associated with and may precede major depressive disorder, manic episodes, etc. allowing for targeted intervention strategies to prevent negative mental illness outcomes), and to determine the factors associated with attention and cognitive disorders (e.g. characterization of sleep loss as a contributing factor to attention-deficit hyperactivity disorder and Alzheimer's disease).

Methylation status of the DMPs and DMP-associated genes described herein may also be beneficial in occupational health and safety applications. For example, identification of people who are sleeping too little to safely perform their required occupational duties (e.g., airplane pilots, bus/train drivers, long haul truckers, etc.), forensic applications (e.g., determining if reduced sleep duration or excessive sleep preceding an accident was a contributing factor in the accident, etc.), and in law-enforcement (e.g., analysis of compliance or non-compliance with fatigued-driver laws where it is a crime to drive if a person has been awake for greater than 24 hours, etc.).

In some embodiments, the methods described herein additionally include a step of treating or providing treatment recommendations to a subject diagnosed with hypersomnolence, idiopathic hypersomnia, or long sleep time. Suitable treatments include administering to a subject a wake promoting medication or stimulant (e.g., amphetamines, modafinil, etc.), administering to a subject an oxybate or derivative thereof (e.g., sodium oxybate), or having the subject undergo sleep extension therapy (e.g., sleeping 1-2 hours longer to see if this improves symptoms). In some embodiments, suitable treatments may include changing one or more sleep practices to improve sleep quality (e.g., amount of light, ambient noise, temperature, etc.), changing the time of day a subject sleeps (e.g., going to bed earlier or staying up later), having a subject nap more or less during the day, or administrating to a subject one or more over the counter sleep promoting aids such as melatonin.

The genomic positions described herein refer to genomic positions as provided in the UCSC hg19 human reference genome (Fujita PA, Rhead B, Zweig AS, Hinrichs AS, Karolchik D, Cline MS, Goldman M, Barber GP, Clawson H, Coelho A, Diekhans M, Dreszer TR, Giardine BM, Harte RA, Hillman-Jackson J, Hsu F, Kirkup V, Kuhn RM, Learned K, Li CH, Meyer LR, Pohl A, Raney BJ, Rosenbloom KR, Smith KE, Haussler D, Kent WJ. The UCSC Genome Browser database: update 2011. Nucleic Acids Res. 2011 Jan;39(Database issue):D876-82. doi: 10.1093/nar/gkq963.) and positions in other genomes aligning thereto. Suitable alignment methods are known in the art. Alignments are typically performed by computer programs that apply various algorithms, however it is also possible to perform an alignment by hand. Alignment programs typically iterate through potential alignments of sequences and score the alignments using substitution tables, employing a variety of strategies to reach a potential optimal alignment score. Commonly-used alignment algorithms include, but are not limited to, CLUSTALW, (see, Thompson J. D., Higgins D. G., Gibson T. J., CLUSTAL W: improving the sensitivity of progressive multiple sequence alignment through sequence weighting, position-specific gap penalties and weight matrix choice, Nucleic Acids Research 22: 4673-4680, 1994); CLUSTALV, (see, Larkin M. A., et al., CLUSTALW2, ClustalW and ClustalX version 2, Bioinformatics 23(21): 2947-2948, 2007); Jotun-Hein, Muscle et al., MUSCLE: a multiple sequence alignment method with reduced time and space complexity, BMC Bioinformatics 5: 113, 2004); Mafft, Kalign, ProbCons, and T-Coffee (see Notredame et al., T-Coffee: A novel method for multiple sequence alignments, Journal of Molecular Biology 302: 205-217, 2000). Exemplary programs that implement one or more of the above algorithms include, but are not limited to MegAlign from DNAStar (DNAStar, Inc. 3801 Regent St. Madison, Wis. 53705), MUSCLE, T-Coffee, CLUSTALX, CLUSTALV, JalView, Phylip, and Discovery Studio from Accelrys (Accelrys, Inc., 10188 Telesis Ct, Suite 100, San Diego, Calif. 92121). In a non-limiting example, MegAlign is used to implement the CLUSTALW alignment algorithm with the following parameters: Gap Penalty 10, Gap Length Penalty 0.20, Delay Divergent Seqs (30%) DNA Transition Weight 0.50, Protein Weight matrix Gonnet Series, DNA Weight Matrix IUB.

The genomic positions of any CpG sites described herein refer to the position of the C in the CpG site in the original genome.

Unless defined otherwise, all technical and scientific terms used herein have the same meaning as commonly understood by one of ordinary skill in the art to which the invention pertains.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein, the terms "approximately" or "about" in reference to a number are generally taken to include numbers that fall within a range of 5% in either direction (greater than or less than) the number unless otherwise stated or otherwise evident from the context (except where such number would exceed 100% of a possible value). Where ranges are stated, the endpoints are included within the range unless otherwise stated or otherwise evident from the context.

### EXAMPLES

### Background

Hypersomnolence, broadly defined as excessive daytime sleepiness often accompanied by prolonged sleep duration, is one of the most common symptom constellations encountered in persons with sleep disorders. While the pathophysiology of hypersomnolence can be identified in some cases, for example orexin deficiency in type 1 narcolepsy,¹ the cause of hypersomnolence cannot universally be determined for many patients despite comprehensive evaluation, resulting in ambiguous diagnoses and a lack of targeted therapies to improve symptomatology. Thus, there is a clear need to better understand the biological basis of unexplained hypersomnolence to advance clinical care through the development of advanced diagnostics and personalized therapeutics for these patients.

One of the great challenges that must be overcome in this area of research is the fact that hypersomnolence is a multifaceted symptom, for which no singular objective measure is able to fully capture the subjective complaint.²⁻³ It is widely appreciated that the multiple sleep latency test (MSLT), while highly useful in confirming type 1 narcolepsy, has limited test-retest reliability in disorders of unexplained hypersomnolence.⁴⁻⁶ While the MSLT remains a key tool in the practice of sleep medicine, it quantifies sleep propensity during repeated nap opportunities and the presence of sleep onset REM periods, but does not capture other relevant aspects of hypersomnolence. Supplementary measures frequently capture other key facets of hypersomnolence, including the maintenance of wakefulness test, which quantifies the ability to remain awake under soporific conditions, infrared pupillometry, which quantifies drowsiness under resting conditions, the psychomotor vigilance task, which quantifies neurobehavioral alertness, and extended duration polysomnographic recordings, which measure excessive sleep duration. While these aspects of hypersomnolence correlate with subjective clinical complaints, they only marginally explain the variance of one another.⁷⁻⁹ Thus, the measurable phenotype in patients with unexplained hypersomnolence can be quite complex, with variable combinations of abnormalities occurring among individual patients.⁷⁻⁹

Since there is heterogeneity among objective measurable deficits in persons with hypersomnolence, it is highly likely that there is also significant heterogeneity in the underlying biology associated with clinical complaints. Sleep medicine has traditionally focused on research that compares various disorders of excessive sleepiness defined by the International Classification of Sleep Disorders (ICSD) both against one another and healthy persons. The diagnostic criteria for disorders such as idiopathic hypersomnia have shifted over time, often with limited clinical or biological data to support alterations in nosology. To disentangle the undoubtedly complex causes of unexplained central nervous system hypersomnolence requires that research efforts focus on the biology of specific and measurable phenotypes that may cut across traditional diagnostic boundaries, rather than nosological distinctions defined largely by expert opinion.¹⁰

The importance of using refined and objective phenotypes is particularly salient for the study of the genetics that may be related to hypersomnolence complaints. Effort has been made to utilize genome-wide association studies (GWAS) to identify candidate single nucleotide polymorphisms (SNPs) that are associated with various sleep phenotypes.¹¹ Unfortunately, the SNPs that are statistically related to sleep measures in these large-scale and hypothesis-free investigations often have modest effect sizes on the overall phenotype, suggesting that genetic variation alone is unlikely to account for extremes of daytime sleepiness and/or sleep duration observed in clinical practice.¹²⁻¹⁶ Additionally, the sleep phenotype used in GWAS studies is almost universally based on self-report, which may be both inaccurate and have limited connection to relevant underlying genetic variability. Thus, while GWAS can help identify potential genes linked to broad phenotypic traits, alternative approaches are needed to connect findings from large-scale population-based GWAS investigations to smaller populations of heterogeneous and disordered patients.

In this context, epigenetics, the study of molecular modifications not affecting the genetic code itself, may be highly applicable to the study of unexplained hypersomnolence. Employing epigenetic methodologies is a crucial step in clarifying how candidate genes are related to specific observable and measurable phenotypes in sleep disorders. In particular, combining epigenetics with a detailed and objective phenotyping approach is likely to advance our understanding of how specific gene regulatory elements are related to the pathophysiology of human disease. Therefore, the present examples examined alterations in DNA methylation, a key component of epigenetic regulation, in a well-characterized sample of patients with unexplained hypersomnolence, to determine if epigenetic modification of candidate genes were related to specific aspects of the hypersomnolence phenotype.

### Methods

### Participants

All participants were clinical patients referred by their treating clinician for sleep testing to evaluate complaints of hypersomnolence at Wisconsin Sleep, the sleep clinic and laboratory affiliated with the University of Wisconsin-Madison. For inclusion, participants had to be 1) free of psychotropic medications at the time of and preceding sleep procedures, 2) not have any identifiable cause of hypersomnolence [*e.g.*, sleep apnea defined as apnea hypopnea index (AHI)>5/hr), evidence of sleep deprivation prior to sleep testing (using either sleep logs and/or actigraphy), complaints of cataplexy suggesting type 1 narcolepsy, etc.], and 3) have no sleep onset REM periods on either overnight polysomnography (PSG) or MSLT. Participants provided informed consent for all study procedures, including both sleep phenotyping measures not part of routine clinical care, as well as collection of biospecimens for subsequent genetic analysis.

### Hypersomnolence Phenotyping Procedures

The hypersomnolence phenotype was determined using four parameters: 1) mean sleep latency on MSLT, 2) total sleep time on ad libitum polysomnography, 3) pupillary unrest index measured by infrared pupillometry, and 4) lapses on the psychomotor vigilance task. All measures were collected within a single sleep laboratory visit.

Participants arrived at the sleep laboratory for testing at approximately 19:00-20:00. After polysomnographic set-up, participants determined their bedtime and were only disturbed after sleep onset if technical issues arose with the recording that would inhibit sleep scoring/staging. The end of the PSG recording was determined by the patient informing the technician that they were ready to get up for the day, rather than a universally applied standard wake time. An MSLT was subsequently performed, with sleep latency defined as the time from lights out to the first 30-second epoch scored as any stage of sleep. The nap was terminated after 20 minutes (if no sleep was achieved) or 15 minutes after the first epoch of scored sleep.¹⁷ Both PSG and MSLT were collected using Alice Sleepware (Phillips Respironics, Murrysville, Pennsylvania, United States) and scored following standard criteria.¹⁸

Following MSLT naps 1 and 3, the pupillographic sleepiness test (PST) and the psychomotor vigilance task were collected with values at each timepoint averaged. The PST is an assessment of drowsiness under constant darkness that records oscillations of the pupil diameter via a computer-based infrared video technique.²⁵ These undulations result from progressive reduction of noradrenergic central activation from the locus coeruleus, resulting in disinhibition of the parasympathetic Edinger-Westphal nucleus.¹⁹ The fluctuations in pupil diameter are utilized to calculate the pupillary unrest index (PUI), defined by absolute values of cumulative changes in pupil size based on the mean values of consecutive data sequences,²⁰ with higher values suggestive of increased drowsiness. Reproducibility, reliability, and normative values of the PUI have been established for adults.^{19,21-23} The PSTEco system (AMTech, Germany) was used for ascertainment of PUI in the present examples and was applied following established protocols.¹⁹

The 10-minute psychomotor vigilance task (PVT) was collected after PST to minimize the potential impact of time-on-task effects from this measure of neurobehavioral alertness on PUI. The PVT is a well-validated measure used in sleep research that quantifies the ability to sustain attention and respond in a timely manner to salient signals.²⁴ The PVT requires responses to a stimulus (digital counter) by pressing a button as soon as the stimulus appears, which stops the stimulus counter and displays the reaction time in milliseconds for a 1-second period. The number of lapses (failure to respond within 500 msec of stimulus; Tukey transformed) was considered the primary PVT measure of interest in the present examples.

### DNA Extraction and Methylation Detection

Saliva samples were collected from participants approximately two hours after awakening using Oragene kits for DNA methylation analysis (DNA Genotek, Canada). DNA was extracted following the manufacturers protocol. Genomic DNA samples were resolved on a 1% agarose gel, to verify the DNA was of high molecular weight, and quantified using Qubit (Qiagen, USA). Genome-wide DNA methylation levels were determined using the HumanMethylationEPIC array and raw intensity data files were imported into the R package *minfi* to assess sample quality, calculate the detection P-value of each tested probe, filter probes, and determine beta values.²⁵ Probes were background- and control-corrected, followed by subset-quantile within array normalization to correct for probe-type bias. ²⁵⁻²⁷ Probes were removed from further analysis if: one sample or more exhibited a detection *P*-value > 0.01; the probe contained a known single nucleotide polymorphism (SNP); the probe was derived from a sex chromosome; the probe measured methylation at a cytosine followed by a nucleotide other than guanine; or the probe was a cross-reactive probe. With these filtration criteria, 97,964 probes were discarded and 768,127 probes were available for further analysis.

### Statistical analyses

Methylation levels (*i.e.,* beta-values) were calculated in *minfi* as the ratio of methylated to total signal (*i.e.*, beta-value = methylated signal/[methylated signal + unmethylated signal + 100]), where beta-values range from 0 (unmethylated) to 1 (methylated). Beta values were further converted to *M*-values (*i.e.,* logit-transformed beta-values) for differential analysis, as *M-*values are more appropriate for statistical testing. Individual models for each independent objective variable of interest [*i.e.,* average PUI on infrared pupillometry, total sleep time (TST) on PSG, mean sleep latency (MSL) on MSLT, and average PVT lapses] were generated, while also accounting for the dependent variables of age, sex, body mass index (BMI), and HumanMethylationEPIC Beadchip identification number. Score on the Epworth Sleepiness Scale (ESS) was similarly evaluated as a subjective measure of daytime sleepiness for comparison purposes.²⁸ Since saliva tissues may be confounded due to latent factors such as heterogeneous cell populations, the R package *sva* was employed to identify any surrogate variables not accounted for in the model.²⁹ In each model, one surrogate variable was identified and adjusted for in the model using *sva.* Linear regression for each tested CpG using a multivariate model was employed using the R package *limma*.³⁰

To assess systematic bias of the linear regression model, the genomic inflation factor was calculated for the obtained *P*-values for each model, yielding a genomic inflation factor of PUI (*λ* = 0.99), TST (*λ* = 1.05), MSL (*λ* = 1.05), PVT (*λ* = 1.37), and ESS (*λ* = 1.19). In the latter two cases (PVT and ESS) the slightly elevated *λ* score suggests modest inflation of the generated P-values, indicative of potential bias in the model. As such, these two models were reanalyzed without adjusting for surrogate variables and the genomic inflation factor was reassessed. Without surrogate variable adjustment, the genomic inflation factors were: PVT (*λ* = 0.956) and ESS (*λ* = 0.76), indicating a more unbiased approach without adjusting for surrogate variables in the cases of the independent variables PVT and ESS. In addition, the R package NHMMfdr used here reports a BIC score based on the *P*-values generated from model fitting for both the adjusted and unadjusted models. Model fitting in NHMMfdr resulted in a lower BIC score using the unadjusted models for PVT and ESS. Together, these data supported that differential methylation modeling should not adjust for the identified surrogate variable for the PVT and ESS variables. Notably, the deflated lambdas for these two cases would result in type II error (i.e., false negative), and may be caused by population stratification in our sample, potentially stemming from the higher proportion of female to male participants.

The methylation levels of immediately flanking probes tested by array-based platforms often exhibit dependence upon one another, and because corrections for multiple testing such as the Benjamini-Hochberg false discovery rate may be inefficient under a varying dependence structure, the R package *NHMMfdr* was used to detect the adjusted local index of significance (aLIS), an extension of adjusted *P*-values, for each probe by first converting all *P*-values to z-scores, followed by employing a Hidden Markov Model to determine the "aLIS *P*-value."³¹ An aLIS *P*-value threshold of <0.05 was used to identify significant differentially methylated loci.

### Results

### Participant Demographics and Phenotype

A summary of the 28 patients included in these analyses is provided in Table 3. The sample was predominantly (75%) female. While the mean age was 31.7±11.9 years, the overall range was relatively broad (20 to 66 years). The mean Epworth Sleepiness Scale score was 12.8±3.6 for the sample. Overall, there also was a wide range of values for each hypersomnolence phenotypic trait of interest (*e.g.,* TST, MSL, PUI, and PVT) across the sample, with minimal correlation between values for any objective measure (FIGS. 1A-1C).

**Table 3: Demographics and Sleep Phenotypic Data for the Sample (N = 28).**

| **Characteristic** | **Value** |
|---|---|
| Female Sex, n (%) | 21 (75) |
| Age in years, mean (SD) | 31.7 (11.9) |
| BMI in kg/m², mean (SD) | 26.2 (5.3) |
| MSLT MSL in minutes (SD) | 12.8 (4.2) |
| PSG TST in minutes (SD) | 521.7 (112.4) |
| PUI (SD) | 5.9 (2.5) |
| Lapses, mean (SD) | 2.5 (1.4) |

### PAX8/PAX8-AS1 DNA methylation levels are associated with sleep duration

We first took a hypothesis-driven approach by examining the DNA methylation levels of eleven genes previously associated with the hypersomnolence phenotype (Table 4). Genomic DNAs from saliva were examined using the HumanMethylationEPIC beadchip array, which provides a quantitative measure of DNA methylation levels at 866,091 CpG/CpH dinucleotides across the genome at single-nucleotide resolution, including enhancers and all coding regions. Initial differential methylation analyses centered only on the 832 CpG sites annotated to these eleven genes and utilized independent regression models of the 5 hypersomnolence scores as the explanatory variable (Methods). These analyses revealed that only 9 CpG sites exhibited significant associations between DNA methylation levels and any of the 5 hypersomnolence measures (Table 1). All 9 differentially methylated CpG sites were annotated to the paired box 8 (*PAX8*) gene and its related antisense gene (*PAX8-AS1*) and these methylation levels were correlated to total sleep time measured using ad libitum PSG (aLIS P-value < 0.05). Among these 9 differentially methylated positions (DMPs) was a notable cluster of 5 CpG sites within a small (~400 base pair) region located in the body of the *PAX8* gene and promoter of *PAX8-AS1* (FIG. 2). All five DMPs within this cluster had a positive correlation (mean 0.31 ± 0.09) with sleep duration (*i.e.*, increasing methylation was associated with longer sleep time). To quantify the magnitude of the effect of methylation of the 5 DMPs, the sample was also stratified into those with TST < and ≥9 hours.^{32,33} Participants with hypersomnia had mean methylation of these 5 DMPs that was 8.6% greater than those without excessive sleep duration (77.4% vs. 68.6%; *P*-value = 0.026; FIG. 3).

**Table 4: A priori list of genes contributing to hypersomnolence.**

| **Gene** | **Name** | **aLIS *P*-value** | **Ref** |
|---|---|---|---|
| *PDE4D* | Phosphodiesterase 4D | *N.S.* | *57,58* |
| *NPR2* | Natriuretic peptide receptor 2 | *N.S.* | *59* |
| *SP140* | Speckled protein 140 | *N.S.* | *59* |
| ***PAX8*** | **Thyroid-specific transcription factor paired box gene 8** | **0.021** | *12,13* |
| *IL1RN* | Interleukin-1 receptor antagonist | *N.S.* | *12,60* |
| *ADORA2A* | Adenosine A(2) receptor | *N.S.* | *61,62* |
| *AR* | Androgen receptor | *N.S.* | *13* |
| *OPHN1* | Oligophrenin-1 | *N.S.* | *13* |
| *PER3* | Period 3 | *N.S.* | *62,63* |
| *CACNA1C* | Calcium voltage-gated channel subunit Alpha1 C | *N.S.* | *64-66* |
| *ZFYVE28* | Zinc Finger FYVE-Type Containing 28 | *N.S.* | *67* |

### Genome-wide DNA methylation levels are associated with other aspects of the hypersomnolence phenotype

We additionally conducted genome-wide exploratory comparisons of the 5 independent continuous variables of hypersomnolence scores as the explanatory variable (Methods), which each yielded a unique set of DMPs. Notably, DMPs were found in two genes. First, *LHX6* contained 11 DMPs associated with mean sleep latency on the MSLT (Table 2). Among these 11 DMPs was a notable cluster of 6 CpG sites within a small (~500 base pair) region that were located in the body of the *LHX6* gene. All six DMPs within this cluster had a negative correlation with MSLT MSL (*i.e.,* increasing methylation was associated with reduced sleep latency). Second, *GABRA2* contained 7 DMPs associated with PUI using infrared pupillometry (Table 2). These 7 DMPs were within a small (~300 base pair) region located in the promoter of the *GABRA2* gene. The majority (6/7) of these DMPs had a negative correlation with sleep duration *(i.e.,* decreasing methylation was associated with increasing PUI). Significant findings were not found using either ESS or PVT as the independent variable in either the hypothesis-driven or genome-wide analyses.

### Discussion

The present examples demonstrate that *PAX8*/*PAX8-AS1* DNA methylation levels are associated with sleep duration quantified using in-laboratory ad libitum polysomnography in a well-characterized, unmedicated group of clinical patients with unexplained hypersomnolence. Specifically, 9 differentially methylated sites within *PAX8*/*PAX8-AS1* were associated with sleep duration, of which 5 formed a distinct cluster of CpG sites within a small (~400 base pair) region in the body of the *PAX8* gene and promoter of *PAX8-AS1.* Since antisense genes frequently play key roles in reducing expression of overlapping sense genes and form self-influencing circuits with regulatory advantages over transcription factor proteins³⁴, the location and clustering of these significantly differentially methylated CpG sites increase the likelihood they may directly impact both *PAX8* and *PAX8-AS1* expression. In fact, more than 70% of the transcripts in humans have antisense transcripts that are generated by independent promoters.³⁵ Antisense transcript can function by the act of its own transcription in *cis* (controlling genes locally on the DNA strand involved in its origination), which regulates sense gene expression by interfering/blocking sense transcriptional machinery.³⁶

While the specific determinants of habitual sleep time remain unknown, recent genome wide association studies (GWAS) have highlighted the role of *PAX8*/*PAX8-AS1* in human sleep duration. *PAX8* likely serves several roles as a master transcription factor crucial to embryonic development and maintenance functions after birth.³⁸ Using both self-report and accelerometer data in population-based cohorts, SNPs in *PAX8*/*PAX8-AS1* have repeatedly demonstrated significant effects on sleep duration.¹²⁻¹⁶ However, the SNPs themselves impact sleep duration only marginally (*i.e.,* only a few minutes longer sleep per day). Thus, while hypothesis-free GWAS investigations have pointed to a role of *PAX8*/*PAX8-AS1* in habitual sleep duration, specific sequence variability in *PAX8*/*PAX8-AS1* is unlikely to be a major contributing factor in pathological hypersomnia. In the present examples, we have further extended these well-replicated associations between *PAX8*/*PAX8-AS1* and sleep duration by finding a significant association between *PAX8*/*PAX8-AS1* DNA methylation levels and nocturnal sleep duration in symptomatic patients. The magnitude of the effect between sleep duration and *PAX8*/*PAX8-AS1* methylation levels *(*e.g., -10%) observed in the present examples is on par with methylation level changes that are the epigenetic hallmark of complex disease phenotypes.³⁹ Unique to our approach was the application of deep phenotyping¹⁰, which carefully and objectively quantified several different facets of hypersomnolence, including ad libitum polysomnography to measure total sleep time.

There are several lines of evidence that further support the role of *PAX8*/*PAX8-AS1,* and particularly its epigenetic modification, on sleep duration and hypersomnia. First, one of the few compounds that has demonstrated a measurable impact on excessive sleep duration in idiopathic hypersomnia is levothyroxine, evaluated in a small open-label study in euthyroid patients.⁴⁰ While *PAX8* is expressed in many tissues, it is predominantly expressed in fetal and adult thyroid tissue, and mutations in *PAX8* have been associated with thyroid dysgenesis and thyroid cancers. Second, fluctuations in cerebral *PAX8* methylation levels mirror patterns of total sleep quantity across the lifespan, with a high rate of change early in the first few years of life, followed by a general plateau beginning in the second decade.⁴¹ Thus, it is probable given the large number of GWAS studies implicating *PAX8*/*PAX8-AS1* in sleep duration, relevant supportive data in the literature, and our findings linking *PAX8*/*PAX8-AS1* methylation to sleep duration, that DNA methylation changes in *PAX8*/*PAX8-AS1* may be a biomarker for, or mechanistically related to hypersomnia.

In the secondary genome-wide analysis, DMPs also were found in two genes that have been associated with hypersomnolence in other investigations, *LHX6* and *GABRA2.* LHX6-positive GABA-releasing neurons in the zona incerta promote NREM sleep, likely by inhibiting hypocretin positive neurons, consistent with a plausible connection between these findings and sleep propensity quantified by the MSLT.⁴⁷ A SNP in *GABRA2* was recently associated with self-reported daytime sleepiness in a large-scale GWAS study, published after the study methods were executed.⁴⁸ Together, these data suggest that genes other than *PAX8* may exhibit disruptions in DNA methylation levels correlated to other aspects of hypersomnolence. Thus, future studies that both replicate the primary finding of an association between PAX8/PAX8-AS1 methylation and sleep duration, as well as associations between LHX6 and MSLT sleep latency and GABRA2 and infrared pupillometry, are indicated.

In summary, the present examples demonstrate a significant association between *PAX8*/*PAX8-AS1* DNA methylation levels and objectively quantified sleep duration in persons with unexplained hypersomnolence. These results represent a key step forward in understanding the underlying biology that may be related to both sleep duration and pathological hypersomnia, bridging key findings in prior GWAS studies to disordered populations in sleep medicine. This work will serve as a nidus for further investigation that clarifies the role of *PAX8*/*PAX8-AS1* regulation and expression in both sleep need and duration for healthy persons and patients with pathological hypersomnia.

### References

1. Dauvilliers Y, Arnulf I, Mignot E. Narcolepsy with cataplexy. Lancet. 2007; 369 (9560): 499-511.
2. Bliwise DL. Is the measurement of sleepiness the Holy Grail of sleep medicine? Am J Respir Crit Care Med. 2001; 163 (7): 1517-1519.
3. Pizza F. Sleepiness Assessment. In: Sleepiness and Human Impact Assessment. Milano, Italy.: Springer; 2014: 313-324.
4. Lopez R, Doukkali A, Barateau L, et al. Test-Retest Reliability of the Multiple Sleep Latency Test in Central Disorders of Hypersomnolence. Sleep. 2017; 40 (12).
5. Ruoff C, Pizza F, Trotti LM, et al. The MSLT is Repeatable in Narcolepsy Type 1 But Not Narcolepsy Type 2: A Retrospective Patient Study. J Clin Sleep Med. 2017.
6. Trotti LM, Staab BA, Rye DB. Test-retest reliability of the multiple sleep latency test in narcolepsy without cataplexy and idiopathic hypersomnia. J Clin Sleep Med. 2013; 9 (8): 789-795.
7. Sangal RB, Thomas L, Mitler MM. Maintenance of wakefulness test and multiple sleep latency test. Measurement of different abilities in patients with sleep disorders. Chest. 1992; 101 (4): 898-902.
8. Johns MW. Sensitivity and specificity of the multiple sleep latency test (MSLT), the maintenance of wakefulness test and the epworth sleepiness scale: failure of the MSLT as a gold standard. J Sleep Res. 2000; 9 (1): 5-11.
9. Plante DT, Cook JD, Prairie ML. Multimodal Assessment Increases Objective Identification of Hypersomnolence in Patients Referred for Multiple Sleep Latency Testing. J Clin Sleep Med. 2020.
10. Robinson PN. Deep phenotyping for precision medicine. Hum Mutat. 2012; 33 (5): 777-780.
11. Raizen DM, Wu MN. Genome-wide association studies of sleep disorders. Chest. 2011; 139 (2): 446-452.
12. Gottlieb DJ, Hek K, Chen TH, et al. Novel loci associated with usual sleep duration: the CHARGE Consortium Genome-Wide Association Study. Mol Psychiatry. 2015; 20 (10): 1232-1239.
13. Lane JM, Liang J, Vlasac I, et al. Genome-wide association analyses of sleep disturbance traits identify new loci and highlight shared genetics with neuropsychiatric and metabolic traits. Nat Genet. 2017; 49 (2): 274-281.
14. Dashti HS, Jones SE, Wood AR, et al. Genome-wide association study identifies genetic loci for self-reported habitual sleep duration supported by accelerometer-derived estimates. Nat Commun. 2019; 10 (1): 1100.
15. Jones SE, Tyrrell J, Wood AR, et al. Genome-Wide Association Analyses in 128,266 Individuals Identifies New Morningness and Sleep Duration Loci. PLoS Genet. 2016; 12 (8): e1006125.
16. Jones SE, van Hees VT, Mazzotti DR, et al. Genetic studies of accelerometer-based sleep measures yield new insights into human sleep behaviour. Nat Commun. 2019; 10 (1): 1585.
17. Littner MR, Kushida C, Wise M, et al. Practice parameters for clinical use of the multiple sleep latency test and the maintenance of wakefulness test. Sleep. 2005; 28 (1): 113-121.
18. Berry R, Brooks R, Gamaldo C, et al. The AASM Manual for the Scoring of Sleep and Associated Events: Rules, Terminology and Technical Specifications, Version 2.0. Darien, Illinois: American Academy of Sleep Medicine; 2012.
19. Eggert T, Sauter C, Popp R, Zeitlhofer J, Danker-Hopfe H, et al. The Pupillographic SleepinessTest in adults: effect of age, gender, and time of day on pupillometric variables. Am J Hum Biol. 2012; 24 (6): 820-828.
20. Lüdtke H, Wilhelm B, Adler M, Schaeffel F, Wilhelm H. Mathematical procedures in data recording and processing of pupillary fatigue waves. Vision Res. 1998; 38 (19): 2889-2896.
21. Wilhelm B, Korner A, Heldmaier K, Moll K, Wilhelm H, Ludtke H. Normative Values of the Pupillographic Sleepiness Test in Male and Female Subjects Aged 20 to 60 Years. Somnologie. 2001; 5: 115-120.
22. Ludtke H, Korner A, Wilhelm B, Wilhelm H. Reproducibility of the pupillographic sleepiness test in healthy men. Somnologie. 2000; 4: 170-172.
23. Wilhelm B, Bittner E, Hofmann A, et al. Short-term reproducibility and variability of the pupillographic sleepiness test. Am J Hum Biol. 2015; 27 (6): 862-866.
24. Dinges DF, Powell JW. Microcomputer analysis of performance on a portable, simple visual RT task sustained operations. Behavioral Research Methods, Instrumentation, and Computers. 1985; 17: 652-655.
25. Aryee MJ, Jaffe AE, Corrada-Bravo H, et al. Minfi: a flexible and comprehensive Bioconductor package for the analysis of Infinium DNA methylation microarrays. Bioinformatics (Oxford, England). 2014; 30 (10): 1363-1369.
26. Maksimovic J, Gordon L, Oshlack A. SWAN: Subset-quantile within array normalization for illumina infinium HumanMethylation450 BeadChips. Genome biology. 2012; 13 (6): R44.
27. Wockner LF, Noble EP, Lawford BR, et al. Genome-wide DNA methylation analysis of human brain tissue from schizophrenia patients. Transl Psychiatry. 2014; 4: e339.
28. Johns MW. A new method for measuring daytime sleepiness: the Epworth sleepiness scale. Sleep. 1991; 14 (6): 540-545.
29. Leek JT, Johnson WE, Parker HS, Jaffe AE, Storey JD. The sva package for removing batch effects and other unwanted variation in high-throughput experiments. Bioinformatics (Oxford, England). 2012; 28 (6): 882-883.
30. Ritchie ME, Phipson B, Wu D, et al. limma powers differential expression analyses for RNA-sequencing and microarray studies. Nucleic acids research. 2015; 43 (7): e47.
31. Kuan PF, Chiang DY. Integrating prior knowledge in multiple testing under dependence with applications to detecting differential DNA methylation. Biometrics. 2012; 68 (3): 774-783.
32. Ohayon MM, Reynolds CF, Dauvilliers Y. Excessive sleep duration and quality of life. Ann Neurol. 2013; 73 (6): 785-794.
33. Lammers GJ, Bassetti CLA, Dolenc-Groselj L, et al. Diagnosis of central disorders of hypersomnolence: A reappraisal by European experts. Sleep Med Rev. 2020; 52: 101306.
34. Pelechano V, Steinmetz LM. Gene regulation by antisense transcription. Nat Rev Genet. 2013; 14 (12): 880-893.
35. Katayama S, Tomaru Y, Kasukawa T, et al. Antisense transcription in the mammalian transcriptome. Science. 2005; 309 (5740): 1564-1566.
36. Hongay CF, Grisafi PL, Galitski T, Fink GR. Antisense transcription controls cell fate in Saccharomyces cerevisiae. Cell. 2006; 127 (4): 735-745.
37. Wang Z, Wilson CM, Mendelev N, et al. Acute and Chronic Molecular Signatures and Associated Symptoms of Blast Exposure in Military Breachers. J Neurotrauma. 2020; 37 (10): 1221-1232.
38. Fernández LP, López-Márquez A, Santisteban P. Thyroid transcription factors in development, differentiation and disease. Nat Rev Endocrinol. 2015; 11 (1): 29-42.
39. Leenen FA, Muller CP, Turner JD. DNA methylation: conducting the orchestra from exposure to phenotype? Clin Epigenetics. 2016; 8: 92.
40. Shinno H, Ishikawa I, Yamanaka M, et al. Effect of levothyroxine on prolonged nocturnal sleep time and excessive daytime somnolence in patients with idiopathic hypersomnia. Sleep Med. 2011; 12 (6): 578-583.
41. Siegmund KD, Connor CM, Campan M, et al. DNA methylation in the human cerebral cortex is dynamically regulated throughout the life span and involves differentiated neurons. PLoS One. 2007; 2 (9): e895.
42. Trivedi MS, Holger D, Bui AT, Craddock TJA, Tartar JL. Short-term sleep deprivation leads to decreased systemic redox metabolites and altered epigenetic status. PLoS One. 2017; 12 (7): e0181978.
43. Nilsson EK, Boström AE, Mwinyi J, Schiöth HB. Epigenomics of Total Acute Sleep Deprivation in Relation to Genome-Wide DNA Methylation Profiles and RNA Expression. OMICS. 2016; 20 (6): 334-342.
44. Vernet C, Arnulf I. Idiopathic hypersomnia with and without long sleep time: a controlled series of 75 patients. Sleep. 2009; 32 (6): 753-759.
45. Evangelista E, Lopez R, Barateau L, et al. Alternative diagnostic criteria for idiopathic hypersomnia: A 32-hour protocol. Ann Neurol. 2018; 83 (2): 235-247.
46. Pizza F, Moghadam KK, Vandi S, et al. Daytime continuous polysomnography predicts MSLT results in hypersomnias of central origin. J Sleep Res. 2013; 22 (1): 32-40.
47. Liu K, Kim J, Kim DW, et al. Lhx6-positive GABA-releasing neurons of the zona incerta promote sleep. Nature. 2017; 548 (7669): 582-587.
48. Wang H, Lane JM, Jones SE, et al. Genome-wide association analysis of self-reported daytime sleepiness identifies 42 loci that suggest biological subtypes. Nat Commun. 2019; 10 (1): 3503.
49. Cortese R, Zhang C, Bao R, et al. DNA Methylation Profiling of Blood Monocytes in Patients With Obesity Hypoventilation Syndrome: Effect of Positive Airway Pressure Treatment. Chest. 2016; 150 (1): 91-101.
50. Ämmälä AJ, Urrila AS, Lahtinen A, et al. Epigenetic dysregulation of genes related to synaptic long-term depression among adolescents with depressive disorder and sleep symptoms. Sleep Med. 2019; 61: 95-103.
51. Alisch RS, Chopra P, Fox AS, et al. Differentially methylated plasticity genes in the amygdala of young primates are linked to anxious temperament, an at risk phenotype for anxiety and depressive disorders. J Neurosci. 2014; 34 (47): 15548-15556.
52. Papale LA, Seltzer LJ, Madrid A, Pollak SD, Alisch RS. Differentially Methylated Genes in Saliva are linked to Childhood Stress. Sci Rep. 2018; 8 (1): 10785.
53. Alisch RS, Van Hulle C, Chopra P, et al. A multi-dimensional characterization of anxiety in monozygotic twin pairs reveals susceptibility loci in humans. Transl Psychiatry. 2017; 7 (12): 1282.
54. Kim H, Dinges DF, Young T. Sleep-disordered breathing and psychomotor vigilance in a community-based sample. Sleep. 2007; 30 (10): 1309-1316.
55. Jansen EC, Dolinoy DC, O'Brien LM, et al. Sleep duration and fragmentation in relation to leukocyte DNA methylation in adolescents. Sleep. 2019; 42 (9).
56. Leu-Semenescu S, Louis P, Arnulf I. Benefits and risk of sodium oxybate in idiopathic hypersomnia versus narcolepsy type 1: a chart review. Sleep Med. 2016; 17: 38-44.
57. Gottlieb DJ, O'Connor GT, Wilk JB. Genome-wide association of sleep and circadian phenotypes. BMC Med Genet. 2007; 8 Suppl 1: S9.
58. Pak VM, Mazzotti DR, Keenan BT, et al. Candidate Gene Analysis in the São Paulo Epidemiologic Sleep Study (EPISONO) Shows an Association of Variant in PDE4D and Sleepiness. Sleep Med. 2018l 47: 106-12.
59. Chen YC, Chen TW, Su MC, et al. Whole Genome DNA Methylation Analysis of Obstructive Sleep Apnea: IL1R2, NPR2, AR, SP140 Methylation and Clinical Phenotype. Sleep. 2016; 39 (4): 743-755.
60. Fang J, Wang Y, Krueger JM. Effects of interleukin-1 beta on sleep are mediated by the type I receptor. Am J Physiol. 1998; 274 (3 Pt 2): R655-660.
61. Bodenmann S, Hohoff C, Freitag C, et al. Polymorphisms of ADORA2A modulate psychomotor vigilance and the effects of caffeine on neurobehavioural performance and sleep EEG after sleep deprivation. Br J Pharmacol. 2012; 165 (6): 1904-1913.
62. Rupp TL, Wesensten NJ, Newman R, Balkin TJ. PER3 and ADORA2A polymorphisms impact neurobehavioral performance during sleep restriction. J Sleep Res. 2013; 22 (2): 160-165.
63. Goel N, Banks S, Mignot E, Dinges DF. PER3 polymorphism predicts cumulative sleep homeostatic but not neurobehavioral changes to chronic partial sleep deprivation. PLoS One. 2009; 4 (6): e5874.
64. Shimada M, Miyagawa T, Kawashima M, et al. An approach based on a genome-wide association study reveals candidate loci for narcolepsy. Hum Genet. 2010; 128 (4): 433-441.
65. Byrne EM, Gehrman PR, Medland SE, et al. A genome-wide association study of sleep habits and insomnia. Am J Med Genet B Neuropsychiatr Genet. 2013; 162B (5): 439-451.
66. Parsons MJ, Lester KJ, Barclay NL, Nolan PM, Eley TC, Gregory AM. Replication of Genome-Wide Association Studies (GWAS) loci for sleep in the British G1219 cohort. Am J Med Genet B Neuropsychiatr Genet. 2013; 162B (5): 431-438.
67. Huang H, Zhu Y, Eliot MN, et al. Combining Human Epigenetics and Sleep Studies in Caenorhabditis elegans: A Cross-Species Approach for Finding Conserved Genes Regulating Sleep. Sleep. 2017; 40 (6).

## Claims

1. A method comprising:
isolating target DNA from a biofluid sample from a subject to obtain isolated target DNA;
treating the isolated target DNA with bisulfite to generate bisulfite modified DNA;
amplifying the bisulfite modified DNA with primers specific to one or more human genomic positions to generate amplified DNA, wherein the one or more human genomic positions align to one or more of positions:
113992930 of chromosome 2 of UCSC hg19 human reference genome,
113993052 of chromosome 2 of UCSC hg19 human reference genome,
113993142 of chromosome 2 of UCSC hg19 human reference genome,
113993304 of chromosome 2 of UCSC hg19 human reference genome,
113993313 of chromosome 2 of UCSC hg19 human reference genome,
113994035 of chromosome 2 of UCSC hg19 human reference genome,
113994578 of chromosome 2 of UCSC hg19 human reference genome,
113995370 of chromosome 2 of UCSC hg19 human reference genome,
113995456 of chromosome 2 of UCSC hg19 human reference genome,
124985406 of chromosome 9 of UCSC hg19 human reference genome,
124985756 of chromosome 9 of UCSC hg19 human reference genome,
124987896 of chromosome 9 of UCSC hg19 human reference genome,
124988720 of chromosome 9 of UCSC hg19 human reference genome,
124989052 of chromosome 9 of UCSC hg19 human reference genome,
124989241 of chromosome 9 of UCSC hg19 human reference genome,
124989294 of chromosome 9 of UCSC hg19 human reference genome,
124989337 of chromosome 9 of UCSC hg19 human reference genome,
124989408 of chromosome 9 of UCSC hg19 human reference genome,
124989550 of chromosome 9 of UCSC hg19 human reference genome,
124989839 of chromosome 9 of UCSC hg19 human reference genome,
46391392 of chromosome 4 of UCSC hg19 human reference genome,
46391436 of chromosome 4 of UCSC hg19 human reference genome,
46391448 of chromosome 4 of UCSC hg19 human reference genome,
46391476 of chromosome 4 of UCSC hg19 human reference genome,
46391524 of chromosome 4 of UCSC hg19 human reference genome,
46391532 of chromosome 4 of UCSC hg19 human reference genome, and
46391694 of chromosome 4 of UCSC hg19 human reference genome; and
quantifying methylation at the one or more human genomic positions in the amplified DNA, wherein the quantifying provides a degree of methylation at the one or more human genomic positions in the amplified DNA, and the degree of methylation at the one or more human genomic positions indicates hypersomnolence in the subject.

2. The method of claim 1, wherein the one or more human genomic positions align to six or more of positions:
113992930 of chromosome 2 of UCSC hg19 human reference genome,
113993052 of chromosome 2 of UCSC hg19 human reference genome,
113993142 of chromosome 2 of UCSC hg19 human reference genome,
113993304 of chromosome 2 of UCSC hg19 human reference genome,
113993313 of chromosome 2 of UCSC hg19 human reference genome,
113994035 of chromosome 2 of UCSC hg19 human reference genome,
113994578 of chromosome 2 of UCSC hg19 human reference genome,
113995370 of chromosome 2 of UCSC hg19 human reference genome,
113995456 of chromosome 2 of UCSC hg19 human reference genome,
124985406 of chromosome 9 of UCSC hg19 human reference genome,
124985756 of chromosome 9 of UCSC hg19 human reference genome,
124987896 of chromosome 9 of UCSC hg19 human reference genome,
124988720 of chromosome 9 of UCSC hg19 human reference genome,
124989052 of chromosome 9 of UCSC hg19 human reference genome,
124989241 of chromosome 9 of UCSC hg19 human reference genome,
124989294 of chromosome 9 of UCSC hg19 human reference genome,
124989337 of chromosome 9 of UCSC hg19 human reference genome,
124989408 of chromosome 9 of UCSC hg19 human reference genome,
124989550 of chromosome 9 of UCSC hg19 human reference genome,
124989839 of chromosome 9 of UCSC hg19 human reference genome,
46391392 of chromosome 4 of UCSC hg19 human reference genome,
46391436 of chromosome 4 of UCSC hg19 human reference genome,
46391448 of chromosome 4 of UCSC hg19 human reference genome,
46391476 of chromosome 4 of UCSC hg19 human reference genome,
46391524 of chromosome 4 of UCSC hg19 human reference genome,
46391532 of chromosome 4 of UCSC hg19 human reference genome, and
46391694 of chromosome 4 of UCSC hg19 human reference genome.

3. The method of claim 1, wherein:
the one or more human genomic positions align to one or more of positions:
113992930 of chromosome 2 of UCSC hg19 human reference genome,
113993052 of chromosome 2 of UCSC hg19 human reference genome,
113993142 of chromosome 2 of UCSC hg19 human reference genome,
113993304 of chromosome 2 of UCSC hg19 human reference genome,
113993313 of chromosome 2 of UCSC hg19 human reference genome,
113994035 of chromosome 2 of UCSC hg19 human reference genome,
113994578 of chromosome 2 of UCSC hg19 human reference genome,
113995370 of chromosome 2 of UCSC hg19 human reference genome, and
113995456 of chromosome 2 of UCSC hg19 human reference genome, and
the degree of methylation at the one or more human genomic positions indicates total sleep time in the subject.

4. The method of claim 1, wherein the one or more human genomic positions align to seven or more of positions:
113992930 of chromosome 2 of UCSC hg19 human reference genome,
113993052 of chromosome 2 of UCSC hg19 human reference genome,
113993142 of chromosome 2 of UCSC hg19 human reference genome,
113993304 of chromosome 2 of UCSC hg19 human reference genome,
113993313 of chromosome 2 of UCSC hg 19 human reference genome,
113994035 of chromosome 2 of UCSC hg19 human reference genome,
113994578 of chromosome 2 of UCSC hg19 human reference genome,
113995370 of chromosome 2 of UCSC hg19 human reference genome, and
113995456 of chromosome 2 of UCSC hg19 human reference genome.

5. The method of claim 1, wherein:
the one or more human genomic positions align to each of positions:
113992930 of chromosome 2 of UCSC hg19 human reference genome,
113993052 of chromosome 2 of UCSC hg19 human reference genome,
113993142 of chromosome 2 of UCSC hg19 human reference genome,
113993304 of chromosome 2 of UCSC hg19 human reference genome,
113993313 of chromosome 2 of UCSC hg19 human reference genome,
113994035 of chromosome 2 of UCSC hg19 human reference genome,
113994578 of chromosome 2 of UCSC hg19 human reference genome,
113995370 of chromosome 2 of UCSC hg19 human reference genome, and
113995456 of chromosome 2 of UCSC hg19 human reference genome; and/or
the one or more human genomic positions align to each of positions:
124985406 of chromosome 9 of UCSC hg19 human reference genome,
124985756 of chromosome 9 of UCSC hg19 human reference genome,
124987896 of chromosome 9 of UCSC hg19 human reference genome,
124988720 of chromosome 9 of UCSC hg19 human reference genome,
124989052 of chromosome 9 of UCSC hg19 human reference genome,
124989241 of chromosome 9 of UCSC hg19 human reference genome,
124989294 of chromosome 9 of UCSC hg19 human reference genome,
124989337 of chromosome 9 of UCSC hg19 human reference genome,
124989408 of chromosome 9 of UCSC hg19 human reference genome,
124989550 of chromosome 9 of UCSC hg19 human reference genome, and
124989839 of chromosome 9 of UCSC hg19 human reference genome; and/or
the one or more human genomic positions align to each of positions:
46391392 of chromosome 4 of UCSC hg19 human reference genome,
46391436 of chromosome 4 of UCSC hg19 human reference genome,
46391448 of chromosome 4 of UCSC hg19 human reference genome,
46391476 of chromosome 4 of UCSC hg19 human reference genome,
46391524 of chromosome 4 of UCSC hg19 human reference genome,
46391532 of chromosome 4 of UCSC hg19 human reference genome, and
46391694 of chromosome 4 of UCSC hg19 human reference genome.

6. The method of any one of claims 1-3, wherein the one or more human genomic positions align to six or more of positions:
124985406 of chromosome 9 of UCSC hg19 human reference genome,
124985756 of chromosome 9 of UCSC hg19 human reference genome,
124987896 of chromosome 9 of UCSC hg19 human reference genome,
124988720 of chromosome 9 of UCSC hg19 human reference genome,
124989052 of chromosome 9 of UCSC hg19 human reference genome,
124989241 of chromosome 9 of UCSC hg19 human reference genome,
124989294 of chromosome 9 of UCSC hg19 human reference genome,
124989337 of chromosome 9 of UCSC hg19 human reference genome,
124989408 of chromosome 9 of UCSC hg19 human reference genome,
124989550 of chromosome 9 of UCSC hg19 human reference genome,
124989839 of chromosome 9 of UCSC hg19 human reference genome,
46391392 of chromosome 4 of UCSC hg19 human reference genome,
46391436 of chromosome 4 of UCSC hg19 human reference genome,
46391448 of chromosome 4 of UCSC hg19 human reference genome,
46391476 of chromosome 4 of UCSC hg19 human reference genome,
46391524 of chromosome 4 of UCSC hg19 human reference genome,
46391532 of chromosome 4 of UCSC hg19 human reference genome, and
46391694 of chromosome 4 of UCSC hg19 human reference genome.

7. The method of any one of claims 1 and 3, wherein the one or more human genomic positions align to one or more of positions:
124985406 of chromosome 9 of UCSC hg19 human reference genome,
124985756 of chromosome 9 of UCSC hg19 human reference genome,
124987896 of chromosome 9 of UCSC hg19 human reference genome,
124988720 of chromosome 9 of UCSC hg19 human reference genome,
124989052 of chromosome 9 of UCSC hg19 human reference genome,
124989241 of chromosome 9 of UCSC hg19 human reference genome,
124989294 of chromosome 9 of UCSC hg19 human reference genome,
124989337 of chromosome 9 of UCSC hg19 human reference genome,
124989408 of chromosome 9 of UCSC hg19 human reference genome,
124989550 of chromosome 9 of UCSC hg19 human reference genome, and
124989839 of chromosome 9 of UCSC hg19 human reference genome.

8. The method of any one of claims 1, 3, and 7, wherein the one or more human genomic positions align to one or more of positions:
46391392 of chromosome 4 of UCSC hg19 human reference genome,
46391436 of chromosome 4 of UCSC hg19 human reference genome,
46391448 of chromosome 4 of UCSC hg19 human reference genome,
46391476 of chromosome 4 of UCSC hg19 human reference genome,
46391524 of chromosome 4 of UCSC hg19 human reference genome,
46391532 of chromosome 4 of UCSC hg19 human reference genome, and
46391694 of chromosome 4 of UCSC hg19 human reference genome.

9. The method of any one of claims 1-8, wherein the primers comprise one or more pairs of primers, each pair of primers amplifying a DNA fragment of 150-350 bp in length.

10. The method of claim 9, wherein the one or more pairs of primers consists of fewer than 10 pairs of primers.

11. The method of any one of claims 1-10, wherein the biofluid is selected from the group consisting of saliva, blood, cerebrospinal fluid, sweat, tears, semen, and urine, optionally wherein the biofluid is blood or saliva.

12. The method of any one of claims 1-11, wherein the subject is a human.

13. The method of any one of claims 1-12, wherein the biofluid sample is from a healthy subj ect.

14. The method of any one of claims 1-12, wherein:
the biofluid sample is from a subject with a mean sleep latency as measured by a multiple sleep latency test of less than 10 minutes; and/or
the biofluid sample is from a subject with a pupillary unrest index as measured by infrared pupillometry of at least 9.8; and/or
the biofluid sample is from a subject demonstrating lapses in a psychomotor vigilance test of at least 4.8.

15. The method of any one of claims 1-12, wherein the biofluid sample is from a subject previously diagnosed with diabetes, asthma, or hypertension.

## Patentansprüche

1. Ein Verfahren, aufweisend:
Isolierung von Ziel-DNA aus einer Bioflüssigkeitsprobe eines Subjekts, um isolierte Ziel-DNA zu erhalten;
Behandlung der isolierten Ziel-DNA mit Bisulfit, um Bisulfit-modifizierte DNA zu erzeugen;
Amplifizieren der Bisulfit-modifizierten DNA mit Primern, die für eine oder mehrere menschliche genomische Positionen spezifisch sind, um amplifizierte DNA zu erzeugen, wobei die eine oder mehreren menschlichen genomischen Positionen mit einer oder mehreren Positionen übereinstimmen:
113992930 von Chromosom 2 des menschlichen Referenzgenoms UCSC hgl9,
113993052 von Chromosom 2 des menschlichen Referenzgenoms UCSC hgl9,
113993142 von Chromosom 2 des menschlichen Referenzgenoms UCSC hgl9,
113993304 von Chromosom 2 des menschlichen Referenzgenoms UCSC hgl9,
113993313 von Chromosom 2 des menschlichen Referenzgenoms UCSC hgl9,
113994035 von Chromosom 2 des menschlichen Referenzgenoms UCSC hgl9,
113994578 von Chromosom 2 des menschlichen Referenzgenoms UCSC hgl9,
113995370 von Chromosom 2 des menschlichen Referenzgenoms UCSC hgl9,
113995456 von Chromosom 2 des menschlichen Referenzgenoms UCSC hgl9,
124985406 von Chromosom 9 des menschlichen Referenzgenoms UCSC hgl9,
124985756 von Chromosom 9 des menschlichen Referenzgenoms UCSC hgl9,
124987896 von Chromosom 9 des menschlichen Referenzgenoms UCSC hgl9,
124988720 von Chromosom 9 des menschlichen Referenzgenoms UCSC hgl9,
124989052 von Chromosom 9 des menschlichen Referenzgenoms UCSC hgl9,
124989241 von Chromosom 9 des menschlichen Referenzgenoms UCSC hgl9,
124989294 von Chromosom 9 des menschlichen Referenzgenoms UCSC hgl9,
124989337 von Chromosom 9 des menschlichen Referenzgenoms UCSC hgl9,
124989408 von Chromosom 9 des menschlichen Referenzgenoms UCSC hgl9,
124989550 von Chromosom 9 des menschlichen Referenzgenoms UCSC hgl9,
124989839 von Chromosom 9 des menschlichen Referenzgenoms UCSC hgl9,
46391392 von Chromosom 4 des menschlichen Referenzgenoms UCSC hgl9,
46391436 von Chromosom 4 des menschlichen Referenzgenoms UCSC hgl9,
46391448 von Chromosom 4 des menschlichen Referenzgenoms UCSC hgl9,
46391476 von Chromosom 4 des menschlichen Referenzgenoms UCSC hgl9,
46391524 von Chromosom 4 des menschlichen Referenzgenoms UCSC hgl9,
46391532 von Chromosom 4 des menschlichen Referenzgenoms UCSC hgl9 und
46391694 von Chromosom 4 des menschlichen Referenzgenoms UCSC hgl9; und
Quantifizierung der Methylierung an der einen oder den mehreren humanen genomischen Positionen in der amplifizierten DNA, wobei die Quantifizierung einen Methylierungsgrad an der einen oder den mehreren humanen genomischen Positionen in der amplifizierten DNA liefert, und der
Methylierungsgrad an der einen oder den mehreren menschlichen genomischen Positionen Hypersomnolenz bei dem Subjekt anzeigt.

2. Verfahren nach Anspruch 1, wobei die eine oder mehrere menschliche genomische Positionen mit sechs oder mehr der Positionen übereinstimmen:
113992930 von Chromosom 2 des menschlichen Referenzgenoms UCSC hgl9,
113993052 von Chromosom 2 des menschlichen Referenzgenoms UCSC hgl9,
113993142 von Chromosom 2 des menschlichen Referenzgenoms UCSC hgl9,
113993304 von Chromosom 2 des menschlichen Referenzgenoms UCSC hgl9,
113993313 von Chromosom 2 des menschlichen Referenzgenoms UCSC hgl9,
113994035 von Chromosom 2 des menschlichen Referenzgenoms UCSC hgl9,
113994578 von Chromosom 2 des menschlichen Referenzgenoms UCSC hgl9,
113995370 von Chromosom 2 des menschlichen Referenzgenoms UCSC hgl9,
113995456 von Chromosom 2 des menschlichen Referenzgenoms UCSC hgl9,
124985406 von Chromosom 9 des menschlichen Referenzgenoms UCSC hgl9,
124985756 von Chromosom 9 des menschlichen Referenzgenoms UCSC hgl9,
124987896 von Chromosom 9 des menschlichen Referenzgenoms UCSC hgl9,
124988720 von Chromosom 9 des menschlichen Referenzgenoms UCSC hgl9,
124989052 von Chromosom 9 des menschlichen Referenzgenoms UCSC hgl9,
124989241 von Chromosom 9 des menschlichen Referenzgenoms UCSC hgl9,
124989294 von Chromosom 9 des menschlichen Referenzgenoms UCSC hgl9,
124989337 von Chromosom 9 des menschlichen Referenzgenoms UCSC hgl9,
124989408 von Chromosom 9 des menschlichen Referenzgenoms UCSC hgl9,
124989550 von Chromosom 9 des menschlichen Referenzgenoms UCSC hgl9,
124989839 von Chromosom 9 des menschlichen Referenzgenoms UCSC hgl9,
46391392 von Chromosom 4 des menschlichen Referenzgenoms UCSC hgl9,
46391436 von Chromosom 4 des menschlichen Referenzgenoms UCSC hgl9,
46391448 von Chromosom 4 des menschlichen Referenzgenoms UCSC hgl9,
46391476 von Chromosom 4 des menschlichen Referenzgenoms UCSC hgl9,
46391524 von Chromosom 4 des menschlichen Referenzgenoms UCSC hgl9,
46391532 von Chromosom 4 des menschlichen Referenzgenoms UCSC hgl9 und
46391694 von Chromosom 4 des menschlichen Referenzgenoms UCSC hgl9.

3. Verfahren nach Anspruch 1, wobei die eine oder mehrere menschliche genomische Positionen mit einer oder mehreren der Positionen übereinstimmen:
113992930 von Chromosom 2 des menschlichen Referenzgenoms UCSC hgl9,
113993052 von Chromosom 2 des menschlichen Referenzgenoms UCSC hgl9,
113993142 von Chromosom 2 des menschlichen Referenzgenoms UCSC hgl9,
113993304 von Chromosom 2 des menschlichen Referenzgenoms UCSC hgl9,
113993313 von Chromosom 2 des menschlichen Referenzgenoms UCSC hgl9,
113994035 von Chromosom 2 des menschlichen Referenzgenoms UCSC hgl9,
113994578 von Chromosom 2 des menschlichen Referenzgenoms UCSC hgl9,
113995370 des Chromosoms 2 des menschlichen Referenzgenoms UCSC hgl9 und
113995456 des Chromosoms 2 des menschlichen Referenzgenoms UCSC hgl9 und der Grad der Methylierung an einer oder mehreren menschlichen genomischen Positionen die Gesamtschlafzeit der Person anzeigt.

4. Verfahren nach Anspruch 1, wobei die eine oder mehrere menschliche genomische Positionen mit sieben oder mehr der Positionen übereinstimmen:
113992930 von Chromosom 2 des menschlichen Referenzgenoms UCSC hgl9,
113993052 von Chromosom 2 des menschlichen Referenzgenoms UCSC hgl9,
113993142 von Chromosom 2 des menschlichen Referenzgenoms UCSC hgl9,
113993304 von Chromosom 2 des menschlichen Referenzgenoms UCSC hgl9,
113993313 von Chromosom 2 des menschlichen Referenzgenoms UCSC hgl9,
113994035 von Chromosom 2 des menschlichen Referenzgenoms UCSC hgl9,
113994578 von Chromosom 2 des menschlichen Referenzgenoms UCSC hgl9,
113995370 des Chromosoms 2 des menschlichen Referenzgenoms UCSC hgl9 und
113995456 von Chromosom 2 des menschlichen Referenzgenoms UCSC hgl9.

5. Verfahren nach Anspruch 1, wobei:
die eine oder mehrere menschliche genomische Positionen mit jeder der Positionen übereinstimmen:
113992930 von Chromosom 2 des menschlichen Referenzgenoms UCSC hgl9,
113993052 von Chromosom 2 des menschlichen Referenzgenoms UCSC hgl9,
113993142 von Chromosom 2 des menschlichen Referenzgenoms UCSC hgl9,
113993304 von Chromosom 2 des menschlichen Referenzgenoms UCSC hgl9,
113993313 von Chromosom 2 des menschlichen Referenzgenoms UCSC hgl9,
113994035 von Chromosom 2 des menschlichen Referenzgenoms UCSC hgl9,
113994578 von Chromosom 2 des menschlichen Referenzgenoms UCSC hgl9,
113995370 des Chromosoms 2 des menschlichen Referenzgenoms UCSC hgl9 und
113995456 von Chromosom 2 des menschlichen Referenzgenoms UCSC hgl9; und/oder
die eine oder mehrere menschliche genomische Positionen mit jeder der Positionen übereinstimmen:
124985406 von Chromosom 9 des menschlichen Referenzgenoms UCSC hgl9,
124985756 von Chromosom 9 des menschlichen Referenzgenoms UCSC hgl9,
124987896 von Chromosom 9 des menschlichen Referenzgenoms UCSC hgl9,
124988720 von Chromosom 9 des menschlichen Referenzgenoms UCSC hgl9,
124989052 von Chromosom 9 des menschlichen Referenzgenoms UCSC hgl9,
124989241 von Chromosom 9 des menschlichen Referenzgenoms UCSC hgl9,
124989294 von Chromosom 9 des menschlichen Referenzgenoms UCSC hgl9,
124989337 von Chromosom 9 des menschlichen Referenzgenoms UCSC hgl9,
124989408 von Chromosom 9 des menschlichen Referenzgenoms UCSC hgl9,
124989550 von Chromosom 9 des menschlichen Referenzgenoms UCSC hgl9 und
124989839 von Chromosom 9 des menschlichen Referenzgenoms UCSC hgl9; und/oder
die eine oder mehrere menschliche genomische Positionen mit jeder der Positionen übereinstimmen:
46391392 von Chromosom 4 des menschlichen Referenzgenoms UCSC hgl9,
46391436 von Chromosom 4 des menschlichen Referenzgenoms UCSC hgl9,
46391448 von Chromosom 4 des menschlichen Referenzgenoms UCSC hgl9,
46391476 von Chromosom 4 des menschlichen Referenzgenoms UCSC hgl9,
46391524 von Chromosom 4 des menschlichen Referenzgenoms UCSC hgl9,
46391532 von Chromosom 4 des menschlichen Referenzgenoms UCSC hgl9 und
46391694 von Chromosom 4 des menschlichen Referenzgenoms UCSC hgl9.

6. Verfahren nach einem der Ansprüche 1 bis 3, wobei die eine oder mehrere menschliche genomische Positionen mit sechs oder mehr der Positionen übereinstimmen:
124985406 von Chromosom 9 des menschlichen Referenzgenoms UCSC hgl9,
124985756 von Chromosom 9 des menschlichen Referenzgenoms UCSC hgl9,
124987896 von Chromosom 9 des menschlichen Referenzgenoms UCSC hgl9,
124988720 von Chromosom 9 des menschlichen Referenzgenoms UCSC hgl9,
124989052 von Chromosom 9 des menschlichen Referenzgenoms UCSC hgl9,
124989241 von Chromosom 9 des menschlichen Referenzgenoms UCSC hgl9,
124989294 von Chromosom 9 des menschlichen Referenzgenoms UCSC hgl9,
124989337 von Chromosom 9 des menschlichen Referenzgenoms UCSC hgl9,
124989408 von Chromosom 9 des menschlichen Referenzgenoms UCSC hgl9,
124989550 von Chromosom 9 des menschlichen Referenzgenoms UCSC hgl9,
124989839 von Chromosom 9 des menschlichen Referenzgenoms UCSC hgl9,
46391392 von Chromosom 4 des menschlichen Referenzgenoms UCSC hgl9,
46391436 von Chromosom 4 des menschlichen Referenzgenoms UCSC hgl9,
46391448 von Chromosom 4 des menschlichen Referenzgenoms UCSC hgl9,
46391476 von Chromosom 4 des menschlichen Referenzgenoms UCSC hgl9,
46391524 von Chromosom 4 des menschlichen Referenzgenoms UCSC hgl9,
46391532 von Chromosom 4 des menschlichen Referenzgenoms UCSC hgl9 und
46391694 von Chromosom 4 des menschlichen Referenzgenoms UCSC hgl9.

7. Verfahren nach einem der Ansprüche 1 und 3, wobei die eine oder mehrere menschliche genomische Positionen mit einer oder mehreren der Positionen übereinstimmen:
124985406 von Chromosom 9 des menschlichen Referenzgenoms UCSC hgl9,
124985756 von Chromosom 9 des menschlichen Referenzgenoms UCSC hgl9,
124987896 von Chromosom 9 des menschlichen Referenzgenoms UCSC hgl9,
124988720 von Chromosom 9 des menschlichen Referenzgenoms UCSC hgl9,
124989052 von Chromosom 9 des menschlichen Referenzgenoms UCSC hgl9,
124989241 von Chromosom 9 des menschlichen Referenzgenoms UCSC hgl9,
124989294 von Chromosom 9 des menschlichen Referenzgenoms UCSC hgl9,
124989337 von Chromosom 9 des menschlichen Referenzgenoms UCSC hgl9,
124989408 von Chromosom 9 des menschlichen Referenzgenoms UCSC hgl9,
124989550 von Chromosom 9 des menschlichen Referenzgenoms UCSC hgl9 und
124989839 von Chromosom 9 des menschlichen Referenzgenoms UCSC hgl9.

8. Verfahren nach einem der Ansprüche 1, 3 und 7, wobei die eine oder mehrere menschliche genomische Positionen mit einer oder mehreren der Positionen übereinstimmen:
46391392 von Chromosom 4 des menschlichen Referenzgenoms UCSC hgl9,
46391436 von Chromosom 4 des menschlichen Referenzgenoms UCSC hgl9,
46391448 von Chromosom 4 des menschlichen Referenzgenoms UCSC hgl9,
46391476 von Chromosom 4 des menschlichen Referenzgenoms UCSC hgl9,
46391524 von Chromosom 4 des menschlichen Referenzgenoms UCSC hgl9,
46391532 von Chromosom 4 des menschlichen Referenzgenoms UCSC hgl9 und
46391694 von Chromosom 4 des menschlichen Referenzgenoms UCSC hgl9.

9. Verfahren nach einem der Ansprüche 1 bis 8, wobei die Primer ein oder mehrere Primerpaare aufweisen, wobei jedes Primerpaar ein DNA-Fragment mit einer Länge von 150-350 bp amplifiziert.

10. Verfahren nach Anspruch 9, wobei das eine oder die mehreren Primerpaare aus weniger als 10 Primerpaaren bestehen.

11. Verfahren nach einem der Ansprüche 1 bis 10, wobei die Bioflüssigkeit aus der Gruppe ausgewählt ist, die aus Speichel, Blut, Liquor, Schweiß, Tränen, Sperma und Urin besteht, wobei es sich bei der Bioflüssigkeit optional um Blut oder Speichel handelt.

12. Verfahren nach einem der Ansprüche 1 bis 11, wobei das Subjekt ein Mensch ist.

13. Verfahren nach einem der Ansprüche 1 bis 12, wobei die Bioflüssigkeitsprobe von einem gesunden Subjekt stammt.

14. Das Verfahren nach einem der Ansprüche 1 bis 12, wobei:
die Bioflüssigkeitsprobe von einem Subjekt stammt mit einer mittleren Schlaflatenz, gemessen durch einen Mehrfach-Schlaflatenztest, von weniger als 10 Minuten; und/oder
die Bioflüssigkeitsprobe von einem Subjekt stammt mit einem Pupillenunruheindex, gemessen durch Infrarot-Pupillometrie von mindestens 9,8; und/oder
die Bioflüssigkeitsprobe von einem Subjekt stammt, die bei einem psychomotorischen Vigilanztest einen Wert von mindestens 4,8 aufweist.

15. Verfahren nach einem der Ansprüche 1 bis 12, wobei die Bioflüssigkeitsprobe von einem Subjekt stammt, bei dem zuvor Diabetes, Asthma oder Bluthochdruck diagnostiziert wurde.

## Revendications

1. - Procédé comprenant :
isoler de l'ADN cible à partir d'un échantillon de fluide biologique provenant d'un sujet pour obtenir de l'ADN cible isolé ;
traiter l'ADN cible isolé avec du bisulfite pour générer de l'ADN modifié au bisulfite ;
amplifier l'ADN modifié au bisulfite avec des amorces spécifiques à une ou plusieurs positions génomiques humaines pour générer de l'ADN amplifié, dans lequel la ou les positions génomiques humaines s'alignent à une ou plusieurs des positions :
113992930 du chromosome 2 du génome humain de référence UCSC hg19,
113993052 du chromosome 2 du génome humain de référence UCSC hg19,
113993142 du chromosome 2 du génome humain de référence UCSC hg19,
113993304 du chromosome 2 du génome humain de référence UCSC hg19,
113993313 du chromosome 2 du génome humain de référence UCSC hg19,
113994035 du chromosome 2 du génome humain de référence UCSC hg19,
113994578 du chromosome 2 du génome humain de référence UCSC hg19,
113995370 du chromosome 2 du génome humain de référence UCSC hg19,
113995456 du chromosome 2 du génome humain de référence UCSC hg19,
124985406 du chromosome 9 du génome humain de référence UCSC hg19,
124985756 du chromosome 9 du génome humain de référence UCSC hg19,
124987896 du chromosome 9 du génome humain de référence UCSC hg19,
124988720 du chromosome 9 du génome humain de référence UCSC hg19,
124989052 du chromosome 9 du génome humain de référence UCSC hg19,
124989241 du chromosome 9 du génome humain de référence UCSC hg19,
124989294 du chromosome 9 du génome humain de référence UCSC hg19,
124989337 du chromosome 9 du génome humain de référence UCSC hg19,
124989408 du chromosome 9 du génome humain de référence UCSC hg19,
124989550 du chromosome 9 du génome humain de référence UCSC hg19,
124989839 du chromosome 9 du génome humain de référence UCSC hg19,
46391392 du chromosome 4 du génome humain de référence UCSC hg19,
46391436 du chromosome 4 du génome humain de référence UCSC hg19,
46391448 du chromosome 4 du génome humain de référence UCSC hg19,
46391476 du chromosome 4 du génome humain de référence UCSC hg19,
46391524 du chromosome 4 du génome humain de référence UCSC hg19,
46391532 du chromosome 4 du génome humain de référence UCSC hg19 et
46391694 du chromosome 4 du génome humain de référence UCSC hg19 ; et
quantifier la méthylation à la ou aux positions génomiques humaines dans l'ADN amplifié, dans lequel la quantification fournit un degré de méthylation à la ou aux positions génomiques humaines dans l'ADN amplifié, et le degré de méthylation à la ou aux positions génomiques humaines indique une hypersomnolence chez le sujet.

2. - Procédé selon la revendication 1, dans lequel la ou les positions génomiques humaines s'alignent à au moins six des positions :
113992930 du chromosome 2 du génome humain de référence UCSC hg19,
113993052 du chromosome 2 du génome humain de référence UCSC hg19,
113993142 du chromosome 2 du génome humain de référence UCSC hg19,
113993304 du chromosome 2 du génome humain de référence UCSC hg19,
113993313 du chromosome 2 du génome humain de référence UCSC hg19,
113994035 du chromosome 2 du génome humain de référence UCSC hg19,
113994578 du chromosome 2 du génome humain de référence UCSC hg19,
113995370 du chromosome 2 du génome humain de référence UCSC hg19,
113995456 du chromosome 2 du génome humain de référence UCSC hg19,
124985406 du chromosome 9 du génome humain de référence UCSC hg19,
124985756 du chromosome 9 du génome humain de référence UCSC hg19,
124987896 du chromosome 9 du génome humain de référence UCSC hg19,
124988720 du chromosome 9 du génome humain de référence UCSC hg19,
124989052 du chromosome 9 du génome humain de référence UCSC hg19,
124989241 du chromosome 9 du génome humain de référence UCSC hg19,
124989294 du chromosome 9 du génome humain de référence UCSC hg19,
124989337 du chromosome 9 du génome humain de référence UCSC hg19,
124989408 du chromosome 9 du génome humain de référence UCSC hg19,
124989550 du chromosome 9 du génome humain de référence UCSC hg19,
124989839 du chromosome 9 du génome humain de référence UCSC hg19,
46391392 du chromosome 4 du génome humain de référence UCSC hg19,
46391436 du chromosome 4 du génome humain de référence UCSC hg19,
46391448 du chromosome 4 du génome humain de référence UCSC hg19,
46391476 du chromosome 4 du génome humain de référence UCSC hg19,
46391524 du chromosome 4 du génome humain de référence UCSC hg19,
46391532 du chromosome 4 du génome humain de référence UCSC hg19, et
46391694 du chromosome 4 du génome humain de référence UCSC hg19.

3. - Procédé selon la revendication 1, dans lequel :
la ou les positions génomiques humaines s'alignent à une ou plusieurs des positions :
113992930 du chromosome 2 du génome humain de référence UCSC hg19,
113993052 du chromosome 2 du génome humain de référence UCSC hg19,
113993142 du chromosome 2 du génome humain de référence UCSC hg19,
113993304 du chromosome 2 du génome humain de référence UCSC hg19,
113993313 du chromosome 2 du génome humain de référence UCSC hg19,
113994035 du chromosome 2 du génome humain de référence UCSC hg19,
113994578 du chromosome 2 du génome humain de référence UCSC hg19,
113995370 du chromosome 2 du génome humain de référence UCSC hg19, et
113995456 du chromosome 2 du génome humain de référence UCSC hg19, et
le degré de méthylation à la ou aux positions génomiques humaines indique le temps de sommeil total chez le sujet.

4. - Procédé selon la revendication 1, dans lequel la ou les positions génomiques humaines s'alignent à au moins sept des positions :
113992930 du chromosome 2 du génome humain de référence UCSC hg19,
113993052 du chromosome 2 du génome humain de référence UCSC hg19,
113993142 du chromosome 2 du génome humain de référence UCSC hg19,
113993304 du chromosome 2 du génome humain de référence UCSC hg19,
113993313 du chromosome 2 du génome humain de référence UCSC hg19,
113994035 du chromosome 2 du génome humain de référence UCSC hg19,
113994578 du chromosome 2 du génome humain de référence UCSC hg19,
113995370 du chromosome 2 du génome humain de référence UCSC hg19, et
113995456 du chromosome 2 du génome humain de référence UCSC hg19.

5. - Procédé selon la revendication 1, dans lequel :
la ou les positions génomiques humaines s'alignent à chacune des positions :
113992930 du chromosome 2 du génome humain de référence UCSC hg19,
113993052 du chromosome 2 du génome humain de référence UCSC hg19,
113993142 du chromosome 2 du génome humain de référence UCSC hg19,
113993304 du chromosome 2 du génome humain de référence UCSC hg19,
113993313 du chromosome 2 du génome humain de référence UCSC hg19,
113994035 du chromosome 2 du génome humain de référence UCSC hg19,
113994578 du chromosome 2 du génome humain de référence UCSC hg19,
113995370 du chromosome 2 du génome humain de référence UCSC hg19, et
113995456 du chromosome 2 du génome humain de référence UCSC hg19 ; et/ou
la ou les positions génomiques humaines s'alignent à chacune des positions :
124985406 du chromosome 9 du génome humain de référence UCSC hg19,
124985756 du chromosome 9 du génome humain de référence UCSC hg19,
124987896 du chromosome 9 du génome humain de référence UCSC hg19,
124988720 du chromosome 9 du génome humain de référence UCSC hg19,
124989052 du chromosome 9 du génome humain de référence UCSC hg19,
124989241 du chromosome 9 du génome humain de référence UCSC hg19,
124989294 du chromosome 9 du génome humain de référence UCSC hg19,
124989337 du chromosome 9 du génome humain de référence UCSC hg19,
124989408 du chromosome 9 du génome humain de référence UCSC hg19,
124989550 du chromosome 9 du génome humain de référence UCSC hg19, et
124989839 du chromosome 9 du génome humain de référence UCSC hg19 ; et/ou
la ou les positions génomiques humaines s'alignent à chacune des positions :
46391392 du chromosome 4 du génome humain de référence UCSC hg19,
46391436 du chromosome 4 du génome humain de référence UCSC hg19,
46391448 du chromosome 4 du génome humain de référence UCSC hg19,
46391476 du chromosome 4 du génome humain de référence UCSC hg19,
46391524 du chromosome 4 du génome humain de référence UCSC hg19,
46391532 du chromosome 4 du génome humain de référence UCSC hg19, et
46391694 du chromosome 4 du génome humain de référence UCSC hg19.

6. - Procédé selon l'une quelconque des revendications 1 à 3, dans lequel la ou les positions génomiques humaines s'alignent à au moins six des positions
124985406 du chromosome 9 du génome humain de référence UCSC hg19,
124985756 du chromosome 9 du génome humain de référence UCSC hg19,
124987896 du chromosome 9 du génome humain de référence UCSC hg19,
124988720 du chromosome 9 du génome humain de référence UCSC hg19,
124989052 du chromosome 9 du génome humain de référence UCSC hg19,
124989241 du chromosome 9 du génome humain de référence UCSC hg19,
124989294 du chromosome 9 du génome humain de référence UCSC hg19,
124989337 du chromosome 9 du génome humain de référence UCSC hg19,
124989408 du chromosome 9 du génome humain de référence UCSC hg19,
124989550 du chromosome 9 du génome humain de référence UCSC hg19,
124989839 du chromosome 9 du génome humain de référence UCSC hg19,
46391392 du chromosome 4 du génome humain de référence UCSC hg19,
46391436 du chromosome 4 du génome humain de référence UCSC hg19,
46391448 du chromosome 4 du génome humain de référence UCSC hg19,
46391476 du chromosome 4 du génome humain de référence UCSC hg19,
46391524 du chromosome 4 du génome humain de référence UCSC hg19,
46391532 du chromosome 4 du génome humain de référence UCSC hg19, et
46391694 du chromosome 4 du génome humain de référence UCSC hg19.

7. - Procédé selon l'une quelconque des revendications 1 et 3, dans lequel la ou les positions génomiques humaines s'alignent à une ou plusieurs des positions :
124985406 du chromosome 9 du génome humain de référence UCSC hg19,
124985756 du chromosome 9 du génome humain de référence UCSC hg19,
124987896 du chromosome 9 du génome humain de référence UCSC hg19,
124988720 du chromosome 9 du génome humain de référence UCSC hg19,
124989052 du chromosome 9 du génome humain de référence UCSC hg19,
124989241 du chromosome 9 du génome humain de référence UCSC hg19,
124989294 du chromosome 9 du génome humain de référence UCSC hg19,
124989337 du chromosome 9 du génome humain de référence UCSC hg19,
124989408 du chromosome 9 du génome humain de référence UCSC hg19,
124989550 du chromosome 9 du génome humain de référence UCSC hg19, et
124989839 du chromosome 9 du génome humain de référence UCSC hg19.

8. - Procédé selon l'une quelconque des revendications 1, 3 et 7, dans lequel la ou les positions génomiques humaines s'alignent à une ou plusieurs des positions :
46391392 du chromosome 4 du génome humain de référence UCSC hg19,
46391436 du chromosome 4 du génome humain de référence UCSC hg19,
46391448 du chromosome 4 du génome humain de référence UCSC hg19,
46391476 du chromosome 4 du génome humain de référence UCSC hg19,
46391524 du chromosome 4 du génome humain de référence UCSC hg19,
46391532 du chromosome 4 du génome humain de référence UCSC hg19, et
46391694 du chromosome 4 du génome humain de référence UCSC hg19.

9. - Procédé selon l'une quelconque des revendications 1 à 8, dans lequel les amorces comprennent une ou plusieurs paires d'amorces, chaque paire d'amorces amplifiant un fragment d'ADN de 150 à 350 pb de longueur.

10. - Procédé selon la revendication 9, dans lequel la ou les paires d'amorces consistent en moins de 10 paires d'amorces.

11. - Procédé selon l'une quelconque des revendications 1 à 10, dans lequel le fluide biologique est choisi dans le groupe consistant en la salive, le sang, le liquide céphalo-rachidien, la sueur, les larmes, le sperme et l'urine, facultativement dans lequel le fluide biologique est du sang ou de la salive.

12. - Procédé selon l'une quelconque des revendications 1 à 11, dans lequel le sujet est un être humain.

13. - Procédé selon l'une quelconque des revendications 1 à 12, dans lequel l'échantillon de fluide biologique provient d'un sujet sain.

14. - Procédé selon l'une quelconque des revendications 1 à 12, dans lequel :
l'échantillon de fluide biologique provient d'un sujet ayant une latence de sommeil moyenne telle que mesurée par un test de latence de sommeil multiple de moins de 10 minutes ; et/ou
l'échantillon de fluide biologique provient d'un sujet avec un indice d'agitation pupillaire tel que mesuré par pupillométrie à infrarouge d'au moins 9,8 ; et/ou
l'échantillon de fluide biologique provient d'un sujet présentant des défaillances dans un test de vigilance psychomotrice d'au moins 4,8.

15. - Procédé selon l'une quelconque des revendications 1 à 12, dans lequel l'échantillon de fluide biologique provient d'un sujet chez qui il a précédemment été diagnostiqué un diabète, de l'asthme ou de l'hypertension.
